# EUROPEAN PATENT APPLICATION

(11) **EP 4 730 350 A1**
(43) Date of publication of application: **22.04.2026**
(21) Application number: 24207106.6
(22) Date of filing: 17.10.2024
(51) Int. Cl.: G16H 20/40, A61B 5/00, G16H 40/63, G16H 20/10

(54) **COMPUTER IMPLEMENTED METHOD, COMPUTER PROGRAM PRODUCT, AND THERAPY DEVICE TO PROVIDE SLEEP DISORDERED BREATHING THERAPY**

(71) Applicant: Koninklijke Philips N.V., 5656 AG Eindhoven (NL)
(72) Inventor: FERREIRA DOS SANTOS DA FONSECA, Pedro Miguel, Eindhoven (NL); CUBA GYLLENSTEN, Illapha Gustav Lars, Eindhoven (NL); HENDRIKS, Cornelis Petrus, 5656AG Eindhoven (NL)
(74) Representative: Philips Intellectual Property & Standards

(57) **Abstract**

There is provided a computer-implemented method for determining a setting of a therapy device adapted to provide sleep disordered breathing (SDB) therapy to a subject. The method comprises receiving medication information representative of medication used by the subject. The method comprises determining trait information based on the medication information. The trait information is representative of an influence of the medication on a pathophysiological trait of the subject. The pathophysiological trait has an influence on the SDB of the subject. The method comprises determining the setting of the therapy device based on the trait information.

## Description

### FIELD OF THE INVENTION

The invention relates to a computer implemented method for determining a setting of a therapy device adapted to provide sleep disordered breathing (SDB) therapy to a subject. Further, the invention relates to a therapy device such as a respiratory support device, a mandibular advancement device and a positional therapy device for providing sleep disordered breathing (SDB) therapy to a subject.

### BACKGROUND OF THE INVENTION

Sleep-disordered breathing (SDB) conditions, such as obstructive sleep apnea (OSA) and central sleep apnea (CSA), are becoming increasingly common, and are particularly prevalent in older people, people with a high body mass index, smokers, heavy drinkers and people with conditions such as coronary artery disease, hypertension and diabetes mellitus.

SDB conditions may be treated in various ways using different therapy devices. One way is to use a therapy device that provides positive airway pressure (PAP) therapy. During PAP therapy pressurized air is provided to a subject to keep the subject's airways open during sleep. When first prescribing PAP therapy, a PAP titration study is carried out for the subject in order to determine a level of airway pressure to be provided to the subject during PAP therapy. Also during the PAP titration study, a suitable PAP therapy modality (e.g. continuous positive airway pressure, CPAP, bilevel positive airway pressure, BiPAP, or automatic positive airway pressure, APAP) and a suitable subject interface (e.g. a nasal pillow, an oronasal/full-face mask) are determined. Another way is to use a positional therapy device for providing positional therapy. The goal of positional therapy is to reduce the time the subject lies in a supine position during sleep. The supine position may negatively affect the airway of the subject. For example, when in a supine position, gravity causes the soft tissue in the airway to obstruct the airway more than in a lateral recumbent position or a prone position. For example, in the supine position, the cross-section of the pharynx is reduced, the upper airway geometry changes unfavorably, and/or the lung volume is reduced. For example, in the supine position, upper airway dilator muscles do not sufficiently keep the upper airway open. So the SDB is reduced by reducing the time in the supine position during sleep. Yet another way is to use a mandibular advancement device (MAD) to provide mandibular advancement. By advancing the mandibular relative to the maxilla, soft tissue and muscles in the upper airway are tightened, which causes a reduced obstruction of the airway during sleep.

Recently, various studies investigated the use of medication to treat SDB, such as Perger, E., Bertoli, S., and Lombardi, C., 2023, "Pharmacotherapy for obstructive sleep apnea: targeting specific pathophysiological traits", Expert Review of Respiratory Medicine, 17:8, 663-673, DOI: 10.1080/17476348.2023.2241353, and Homer, R.L., 2023, "Targets for obstructive sleep apnea pharmacotherapy: principles, approaches, and emerging strategies", Expert Opinion on Therapeutic Targets, 27:7, 609-626, DOI: 10.1080/14728222.2023.2240018.

### SUMMARY OF THE INVENTION

The studies indicate that medication can be used to treat the severity of the SDB to a certain extent, causing a reduction of the number and severity of SDB events. However, the medication does not remove all symptoms of the SDB. Therefore, a subject using medication to treat SDB will likely still need to use a therapy device to further reduce the number and severity of SDB events.

It is an objective of the invention to provide an improved SDB therapy with the therapy device for a subject using medication for treating the SDB.

According to a first specific aspect, there is provided a computer-implemented method for determining a setting of a therapy device adapted to provide sleep disordered breathing (SDB) therapy to a subject. The method comprises receiving medication information representative of medication used by the subject. The method comprises determining trait information based on the medication information. The trait information is representative of an influence of the medication on a pathophysiological trait of the subject. The pathophysiological trait has an influence on the SDB of the subject. The method comprises determining the setting of the therapy device based on the trait information.

The pathophysiological trait has an influence on SDB. For example, the pathophysiological trait is the main cause for the SDB, or is one of several causes for the SDB. For example, the pathophysiological trait worsens the interruption or limitation of breath during sleep. As the medication has an influence on the pathophysiological trait, the medication influences the SDB. By determining the trait information, it is determined how the medication influences the pathophysiological trait. For example, the trait information indicates how much the medication improves the pathophysiological trait. By making use of the trait information, the setting of the therapy device is improved. With the improved setting of the therapy device, the therapy device provides improved SDB therapy to the subject.

For example, the therapy device is respiratory support device, such as a positive airway pressure (PAP) therapy device. Examples of a PAP therapy device are a continuous PAP (CPAP) therapy device, a bi-level PAP (BiPAP) therapy device, or automatic PAP (AutoPAP) therapy device. The CPAP therapy device is adapted to provide pressurized air to the airway of the subject at a constant pressure level. The BiPAP therapy device is adapted to provide pressurized air to the airway of the subject at an inhalation pressure level and an exhalation pressure level that is different than the inhalation pressure level. The AutoPAP therapy device is adapted to provide pressurized air to the airway of the subject with a variable pressure within a pressure range.

For example, the therapy device is a positional therapy device. Positional therapy is aimed at preventing or reducing the subject to lie in a target position that worsens or causes the SDB. Typically, the target position is the supine position, in which the subject is lying on the back. In the supine position, one or more of the causes mentioned in the background may cause partial or complete obstruction of the airway. The positional therapy device has a body position sensor adapted to generate a positional signal representative of a body position of the subject. If the positional signal indicates the subject is in the supine position, the positional therapy device causes a stimulus to be provided to the subject. The stimulus prompts the subject to change to a different position, away from the supine position.

For example, the therapy device is a mandibular advancement device (MAD). The MAD causes the lower jaw, the mandible, to be advanced relative to the upper jaw, the maxilla. Because of this advancement, soft tissue and muscles in the upper airway are tightened. The tightening of the soft tissue and muscles causes a reduced obstruction of the airway during sleep.

The trait information comprises, for example, information about a presence and/or a severity of a pathophysiological trait of the subject that has an influence on the SDB. For example, pathophysiological trait is a condition that at least partly contributes to the SDB. For example, the pathophysiological trait is one or more of a high pharyngeal collapsibility, a high loop gain, a low muscle responsiveness, and a low arousal threshold. For example, the pathophysiological trait causes or worsens an obstruction of the airway of the subject during sleep. For example, the pathophysiological trait causes or worsens a ventilatory drive or respiratory drive of the subject during sleep. For example, the trait information comprises information about the influence of the medication varying over a single sleep session or varying over multiple sleep sessions. For example, the trait information comprises information about the influence of the medication based only on the medication information, or on a combination of medication information and at least one measurement of a physiological parameter of the subject. The physiological parameter is representative of an influence of the medication on the subject.

The setting controls, for example, a start and/or an end of the SDB therapy provided by the therapy device. The setting relates, for example, to an intensity level of the SDB therapy, or to a comfort setting, such as the humidification or temperature of a pressurized airflow provided to the subject.

In case the therapy device is the respiratory support device, the setting of the therapy device is, for example, a pressure setting controlling pressure of the pressurized airflow to the airway of the subject. For example, the pressure setting controls a maximum pressure of the pressurized airflow, or a minimum pressure of the pressurized airflow, or a pressure profile over time of the pressurized airflow. For example, the pressure setting controls an increase or a decrease of the pressure of the pressurized airflow over a time period.

In case the therapy device is the positional therapy device, the setting of the therapy device is, for example, an intensity or a duration of the stimulus provided by the positional therapy device. For example, the setting indicates a range of positions that causes the positional therapy device to provide the stimulus. The range of positions may include the supine position. For example, the range of positions includes the supine position and positions less than 90° relative to the supine position, such as less than 45° or less than 30° relative to the supine position. The position at 90° relative to the supine position is a lateral recumbent position. For example, the setting indicates a delay between determining that the subject is in the supine position and providing the stimulus.

In case the therapy device is the MAD, the setting is, for example, the advancement, i.e., the offset, between the mandible and the maxilla as controlled by the MAD. For example, the setting is an advancement profile during the sleep session, wherein the advancement changes during the sleep session.

In an embodiment, the medication information comprises information about a half-life of the medication, or a dosage of the medication, or a tolerance to the medication, or an effectiveness of the medication, or a duration of use of the medication, or a pharmacokinetic (PK) model, or a pharmacodynamic (PD) model, or a pharmacokinetic/ pharmacodynamic (PK/PD) model.

According to the embodiment, the trait information comprises information about the influence of the medication on the pathophysiological trait. By using this information, it can be estimated how severe the SDB of the subject is. For example, in case the medication has a high influence on the pathophysiological trait, the severity of the SDB is lower than when the medication has a low influence on the pathophysiological trait. By using the trait information, the setting of the therapy device is set to provide appropriate therapy by the therapy device in view of the severity of the SDB.

The half-life of the medication is the time it takes for a concentration of the medication in the body of the subject to reduce from a maximum concentration to 50% of that maximum concentration. So when the subject administers the medication, it takes some time for the medication to reach the maximum concentration in the body of the subject. After reaching the maximum concentration, the concentration reduces till it reaches 50% of that maximum concentration. As the half-life is an indicator of the concentration of the medication over time, the half-life provides information about the influence of the medication on the pathophysiological trait. The medication may still have some therapeutic effect at the half-life, or may have no more therapeutic effect if the concentration at the half-life is too low to provide any therapeutic effect. For example, the half-life of acetazolamide is 2.4 - 5.8 hours. For example, the half-life of sulthiame is 24 hours. For example, the half-life of reboxetine and oxybutynin is about 12 hours.

The dosage of the medication indicates the amount of the medication administered to the subject over a certain period. For example, the dosage is representative of the medication taken during a day or during several days or during a week or during a month. The dosage includes, for example, the number of pills with the medication taken by the subject. The dosage includes, for example, the dose with which the medication is taken.

The information about the tolerance includes, for example, information about a reduced effectiveness of the medication after a period of using the medication. For some medications, if the subject uses the medication for a certain period, for example for more than a week or more than a month of more than a year, the medication becomes less effective. For example, the subject uses one or more other medications that cause the subject to be more tolerant to the medication that influences the pathophysiological trait. For example, the information includes information about the tolerance dependent on age, gender, and/or ethnicity. Because of the tolerance, the medication has less influence on the pathophysiological trait. For example, some hypnotics are known to cause tolerance quickly. As a result, those hypnotics stop providing a therapeutic effect after about 2 or 3 weeks of use.

The duration of use of the medication is, for example, a period during which the subject takes the medication as prescribed. The duration of use includes or excludes a period during which the subject temporarily interrupts taking the medication. For example, the duration of use is limited to the week before, or the month before, or the year before determining the setting of the therapy device. For example, the duration of use causes the subject to become more sensitive to the medication. For example, the duration of use causes the subject to become more tolerant to the medication. For example, the duration of use relates to a build-up of the concentration of the medication in the body of the subject.

The information about the effectiveness of the medication includes, for example, information about the influence of the medication on the pathophysiological trait during a sleep session, or during a day or during a longer period, such as a week or a month. The information about the effectiveness of the medication is, for example, based on the half-life and/or the dosage and/or the tolerance information and/or the duration of use.

A pharmacokinetic (PK) model simulates the plasma concentration of the medication as a function of time, depending on medication parameters such as dose, absorption rate constant, volume of distribution (usually reported as liter / kg body weight), elimination rate constant (related to drug half-life), and bioavailability (such as fraction of drug that reaches the systemic circulation after administration). Pharmacokinetic parameter values can be found in clinical literature and databases for many medications. Sophisticated pharmacokinetic models are available from textbooks to calculate the plasma concentration due to repeated dose administration. For example, such a sophisticated pharmacokinetic model simulates a plasma concentration profile for multiple oral dose administrations.

A pharmacodynamic (PD) model calculates the therapeutic effect as a function of plasma concentration of the medication. The pharmacodynamic model represents, for example, different behaviors assuming specific relationships between medication concentrations and effects, e.g. linear, logarithmic, or sigmoid.

A pharmacokinetic-pharmacodynamic (PKPD) model combines the PK model to simulate the plasma concentration of the medication with the PD model that calculates the therapeutic effect as a function of the plasma concentration. The PKPD model takes, for example, into account the patient weight, dose and prescribed timing of intake, to provide a more accurate estimation of the physiological effects over time. PKPD modeling is applied in drug development, clinical pharma studies and in complex hospital environments where individualized dosing is important, e.g., surgical ICU. The PKPD model may include the influence of food intake, body position and obesity.

PK models, PD models, and PKPD models are well-known for medications, such as described in the article by Gerlach, A.T., and Saliba, L., 2016, "Practical Pharmacokinetics and Pharmacodynamics". In: Martin, N.D., Kaplan, L.J. (eds) Principles of Adult Surgical Critical Care. Springer, Cham. https://doi.org/10.1007/978-3-319-33341-0_41. By making use of the information from such models, the influence of the medication on the pathophysiological trait is estimated. This way, an improved setting for the therapy device is obtained.

In an embodiment, the method comprises determining trait information during a first time period and during a second time period. During the first time period the influence of the medication on the pathophysiological trait is larger than during the second time period. The method comprises determining the setting of the therapy device to provide the SDB therapy having a therapy parameter. The therapy parameter has a first value during the first time period and has a second value during the second time period. The first value is different from the second value.

According to this embodiment, during the first time period, the medication has a larger influence on the pathophysiological trait than during the second time period. As a result of this larger influence, the SDB is less severe during the first time period. As a result, the setting of the therapy device is set to provide the SDB therapy having the first value instead of the second value. During the second time period, the medication has less influence than during the first time period. For example, the medication was not yet able to cause the therapeutic effect, or the medication is past the peak of the therapeutic effect. As a result, the SDB is worse during the second time period than during the first time period. The setting of the therapy device is set to provide the SDB therapy with the therapy parameter having the second value instead of the first value. For example, the first time period is the period before the half-life of the medication, whereas the second time period is after the half-life of the medication. For example, during the first time period, the influence of the medication on the pathophysiological trait is above a threshold level. During the second time period, the influence of the medication on the pathophysiological trait is below the threshold level. The second time period is, for example, before the first time period.

In an embodiment, the therapy parameter relates to at least one of: an intensity level of the SDB therapy, or a rate of change of the intensity level of the SDB therapy, or a range of the intensity level of the SDB therapy, or a difference in intensity level of the SDB therapy between inhalation and exhalation of the subject.

During the second time period, the SDB is worse than during the first time period. During the first time period, the setting is, for example, set at a lower intensity level, because the lower intensity level is sufficient to properly treat the less severe SDB. The lower intensity level improves comfort to the subject. The improved comfort helps the subject to adhere to the SDB therapy. For example, the lower intensity level is zero, meaning that no SDB therapy is provided during the first time period by the therapy device. During the second time period, the setting is, for example, set at the higher intensity level, to properly treat the more severe SDB. For example, during the first time period, the intensity level of the SDB therapy changes slowly from a low intensity level to a high intensity level. Because of the slow change, the subject has more time to get used to the increasing intensity level. As a result, the subject has less arousals and/or less awakenings during the sleep session. The low intensity level of the SDB therapy provides sufficient treatment during the first time period. For example, during the second time period, the intensity level of the SDB therapy changes fast from the low intensity level to the high intensity level. Because of the fast change, the subject receives high intensity SDB therapy quickly to properly treat the severe SDB. For example, the therapy device is able to provide the SDB therapy within a range of intensity levels. The setting causes, for example, the therapy device to use a different range of the intensity level for the first time period than for the second time period. For example, during the second time period, the range has a higher maximum value, or a higher minimum value, or a lower minimum value, than during the first time period. For example, an APAP therapy provides a pressurized airflow with a variable pressure in a first pressure range during the first time period, and with a second pressure range during the second time period. The first pressure range is different from the second pressure range. The intensity level is, for example, based on the pressure of the pressurized airflow from the respiratory support device to the subject, the intensity of the stimulation by the positional therapy device, or the advancement of the MAD.

In an embodiment, the method comprises adjusting the therapy device according to the setting to provide the SDB therapy having the first value during the first time period and having the second value during the second time period.

According to this embodiment, a signal is provided to the therapy device to adjust the SDB therapy according to the first value during the first time period and according to the second value during the second time period. Based on this signal, the therapy device is, for example, adapted to adjust the intensity level. For example, based on the signal, a pressure source of the respiratory support device changes the pressure of the pressurized airflow. For example, based on the signal, a controller of the positional therapy device changes the intensity level of the stimulation. For example, based on the signal, an actuator of the MAD changes the advancement.

In an embodiment, the first time period and the second time period are in a same sleep session of the subject, or the first time period and the second time period are in different sleep sessions of the subject.

The first time period and the second time period may be in the same sleep session. During this sleep session, the second time period is, for example, the time between the subject going to bed and the medication starting to have the therapeutic effect. It may take some time before the medication starts to have the therapeutic effect, after the subject has taken the medication. This may be several minutes, or 1 or 2 hours. During this sleep session, the second time period is, for example, the time after the medication had the therapeutic effect. The medication may not have the therapeutic effect long enough to cover the entire sleep session. During the first time period, the medication has the therapeutic effect. In another example, the medication has some therapeutic effect during the second time period, and a larger therapeutic effect during the first time period.

The first time period and the second time period may be in the different sleep sessions. For example, the second time period includes when the subject has just started using the medication. Some medications have to be taken for a certain duration to build up a sufficient concentration to provide the therapeutic effect. The subject may need to take the medication a certain amount of time to become more sensitive to the effect of the medication. For example, the second time period includes when the subject has been using the medication for a prolonged period and the subject has become tolerant to the medication. The first time period is when the medication has a larger influence on the pathophysiological trait than during the second time period. In another example, the medication has some therapeutic effect during the second time period, and a larger therapeutic effect during the first time period.

### TOLERANCE

In an embodiment, the method comprises: receiving patient information relating to a tolerance of the medication; determining a tolerance to the medication based on the patient information; and determining the setting of the therapy device based on the tolerance to the medication.

According to this embodiment, the way the subject develops the tolerance to the medication depends on specifics of the patient. For example, the subject has a comorbidity, such as a kidney condition, that affects the tolerance. For example, the subject has a history of building a tolerance to the medication. Determining the tolerance while using the medication may be based on how the tolerance built up in the past. For example, the subject has a slow metabolism, a normal metabolism, or a fast metabolism. The speed of the metabolism may affect tolerance build up. By taking such information into account, the tolerance to the medication is determined to determine the setting of the therapy device.

In an embodiment, the patient information comprises comorbidity information representative of a drug metabolism of the subject affected by a comorbidity of the subject. The method comprises determining the tolerance to the medication based on the comorbidity information.

According to this embodiment, the tolerance to the medication is based on the comorbidity information. The subject has, next to SDB, a comorbidity. The comorbidity relates, for example, to organs that breakdown or metabolize medication, such as liver, kidneys, and pancreas. The comorbidity relates to, for example, diabetes or chronic kidney disease. So a subject with a liver condition, a kidney condition or a pancreas condition may breakdown or metabolize the medication differently than subjects without such a condition. The way the medication is absorbed by the body influences the buildup of the tolerance to the medication. By making use of the comorbidity information, the tolerance to the medication is determined.

In an embodiment, the patient information comprises drug-drug interaction information representative of a drug metabolism of the subject affected by an interaction of the medication and a further medication used by the subject. The method comprises determining the tolerance to the medication based on the drug-drug interaction information.

According to this embodiment, the tolerance to the medication is based on the drug-drug interaction information. The subject uses the medication and a further medication. The drug-drug interaction information comprises information about how the medication and a further medication influence the drug metabolism of the subject. The drug metabolism is the metabolic breakdown of the medication by the subject. The drug metabolism determines, for example, how fast the body of the subject breaks down the medication. The way the medication is broken down by the body influences the buildup of the tolerance to the medication. By making use of the drug-drug interaction information, the tolerance to the medication is determined.

In an embodiment, the method comprises receiving further medication information representative of further medication used by the subject. The further medication does not influence the pathophysiological trait of the subject. The further medication information comprises information of a tolerance to the further medication. The method comprises determining the tolerance to the medication based on the information of a tolerance to the further medication.

According to this embodiment, information about the tolerance of the further medication is available, for example, via a public database, or the patient's health record, or via testing of the subject. The tolerance of the further medication has a relation with the tolerance of the medication that has an influence on the SDB. For example, the both the medication and the further medication are of the same class, or have the same working mechanism, or act on the some of the same organs, or have some of the same side effects. For example, it is known that the further medication causes a tolerance after several weeks of use, such as 4 weeks or 8 weeks or 12 weeks or 26 weeks or 52 weeks, which makes the further medication less effective by 10% or by 20% or by 25% or by 50% or by more than 50%. Based on this information, the tolerance to the medication is determined. The tolerance is, for example, exactly the same as the tolerance to the further medication. The setting of the therapy is determined to compensate for the tolerance to the medication, for example, to increase the intensity level to compensate for the tolerance to the medication.

In an embodiment, the further medication information comprises information of the tolerance of the subject to the further medication.

According to this embodiment, the further medication information comprises personal information of the subject in view of the tolerance to the further medication. For example, the further medication information comprises information about the use and the effectiveness of the further medication by the subject. For example, the further medication information comprises information about one or more tests conducted on the subject. For example, the concentration of the further medication during use of the further medication by the subject is tested. For example, a physiological property during use of the further medication by the subject is tested.

In an embodiment, the further medication information comprises information of the tolerance of a group of subjects to the further medication.

According to this embodiment, the further medication information comprises information of a group of subjects in view of the tolerance to the further medication. For example, the further medication information comprises information about the use and the effectiveness of the further medication by the subjects in the group. For example, the further medication information comprises information about one or more tests conducted on the subjects in the group. For example, the concentration of the further medication during use of the further medication by the subjects in the group is tested. For example, a physiological property during use of the further medication by the subjects in the group is tested. The group of subjects is, for example, a group of at least 10 subjects, or at least 50 subjects, or at least 100 subjects.

In an embodiment, the medication information comprises information of the tolerance of a group of subjects to the medication.

In an embodiment, the medication and the further medication are in the same category.

Medications can be grouped into categories based on the mechanism of action. The mechanism of action causes the pharmacological and/or physiological effects of the medication. For example, the US Food and Drug Administration (FDA) defines a category of antidepressants. All the antidepressants in this category have as the mechanism of action the ability to improve monoaminergic transmission. Because of the same mechanism of action, a subject would build up tolerance to any one of the antidepressants in the category in a similar way. The category of antidepressants has multiple classes. One of the classes is serotonin-norepinephrine reuptake inhibitors (SNRIs). The medication may comprise an SNRI to treat SDB. As the SNRIs has the same mechanism of action as the further medications in the category, tolerance information about the further medication can be used to determine the tolerance of the medication.

### METHOD RELATING MAD

In an embodiment, the therapy device comprises a mandibular advancement device (MAD). The MAD comprises a first member, a second member, and an adjustment device. The first member is adapted to engage a maxilla of the subject. The second member is adapted to engage a mandible of the subject. The adjustment device is adapted to adjust an offset between the first member and the second member to advance the mandible relative to the maxilla. The method comprises generating an instruction signal representative of an instruction for adjusting the offset with the adjustment device. The instruction signal is based on determining the setting of the therapy device based on the trait information.

By adjusting the offset between the first member and the second member, the intensity of the mandibular advancement therapy provided by the MAD is adjusted. In case the medication has a large influence on the pathophysiological trait, a small offset may be sufficient to provide effective therapy. In case the medication has a small influence on the pathophysiological trait, a large offset may be needed to provide effective therapy. However, the large offset causes discomfort to the subject, and may cause the subject to sleep poorly or to not adhere to using the MAD as prescribed by the health professional. The instruction signal provides an instruction to adjust the offset of the MAD with the adjustment device. Because the instruction signal is based on the setting of the therapy device based on the trait information, the instruction signal takes into account the influence of the medication on the SDB. When the medication has a large positive influence on the SDB, the instruction signal indicates to adjust the offset of the MAD to a small offset, whereas when the medication only has a small influence or no influence on the SDB, the instruction signal indicates to adjust the offset of the MAD to a large offset. As a result, improved SDB therapy is provided with the therapy device for the subject using medication for treating the SDB.

For example, based on the instruction signal, the subject using the MAD is able to adjust to a small offset during the first time period during which the medication has a large influence on the pathophysiological trait. Based on the instruction signal, the subject using the MAD is able to adjust to a large offset during the second time period during which the medication has a small or no influence on the pathophysiological trait. For example, the second time period is when the subject had just started taking the medication, and the medication concentration needs to build up over several days or weeks to have a therapeutic effect. For example, the second time period is when the subject has been taken the medication for a prolonged time, such as several months or years, and a tolerance to the medication has been built up. For example, the first time period is when the medication achieves the desired therapeutic effect.

In an embodiment, the adjustment device comprises an actuator. The method comprises providing the instruction signal to the actuator. The actuator is adapted to adjust the offset between the first member and the second member based on the instruction signal.

Based on the instruction signal, the actuator is adapted to adjust the offset of the MAD. This allows adjustment of the offset without the need of manual intervention by the subject. This prevents the subject from applying the wrong offset in response to the instruction signal. Further, in case a single sleep session has the first time period and the second time period, the actuator is able to adjust the offset during the sleep session. For example, the second time period is a time between when the subject administers the medication before the sleep session and when the medication has its therapeutic effect. For example, the medication may not have the therapeutic effect long enough to cover the entire sleep session. The second time period is the part of the sleep session during which the medication provides only a subtherapeutic effect or no more therapeutic effect.

### INTRODUCTION FOUR PATHOPHYSIOLOGICAL TRAITS

The study of Perger et.al, as mentioned in the background, indicates four pathophysiological traits that have an influence on SDB. These four pathophysiological traits are a high pharyngeal collapsibility, a high loop gain, a low muscle responsiveness, and a low arousal threshold. The study indicates that medication is available for each of the four pathophysiological traits. Medication is available that has a mechanism of action that predominantly targets one or more of these four pathophysiological traits. The following aspects of the invention, i.e., the second aspect to the fifth aspect, make use of the insights by the inventors that by using the trait information representative of an influence of the medication on one of these four pathophysiological traits, an improved setting of the therapy device is obtained to provide improved SDB therapy to the subject who uses medication that targets that pathophysiological trait.

### HIGH PHARYNGEAL COLLAPSIBILITY

According to a second aspect of the invention, there is provided a method according to the first aspect, wherein the pathophysiological trait comprises a high pharyngeal collapsibility.

Increased adiposity and/or edema can cause the airway to be narrowed and can cause additional pressure by soft tissue in the upper airway. This causes the pharynx of the subject to collapse during sleep, and to either partly or completely close the airway. The collapsibility of the pharynx of the subject is thus higher than of a healthy subject. When the pharynx collapses or narrows during sleep, the subject has a higher risk for an SDB event, such as an apnea event or a hypopnea event.

As a result, the high pharyngeal collapsibility has an influence on the SDB of the subject. The subject uses one or more medications that influence the high pharyngeal collapsibility. For example, the medication reduces the water retention in the body. For example, the medication is a diuretic to reduce water retention. For example, the medication prevents or reduces fluid shift from the legs of the subject towards the airway. For example, the medication comprises spironolactone or furosemide.

For example, the medication reduces body fat in the body. As a result of reducing body fat, body fat around the upper airway is reduced. For example, the medication to reduce body fat comprises a GLP1-agonist, an STGLT2-inhibtor, or a lipases inhibitor. For example, the medication comprises liraglutide, semaglutide, naltrexone and bupropion, or orlistat.

As the medication has an influence on the high pharyngeal collapsibility, the medication influences the SDB. By making use of the trait information about the high pharyngeal collapsibility, the setting of the therapy device is improved. With the improved setting of the therapy device, the therapy device provides improved SDB therapy to the subject.

In an embodiment, the invention according to the second aspect is combined with any of the embodiments according to the first aspect of the invention.

In an embodiment, the medication comprises a diuretic. The method comprises estimating a fluid shift in the subject during a sleep session caused by the diuretic based on the medication information. The method comprises determining the trait information based on the estimated fluid shift.

The subject may suffer from excessive cellular fluid accumulation and/or edema. This increase in fluid in the body can worsen SDB by thickening the tissues in the upper airway and increasing the mass against which the upper airway muscles need to press. Several diseases, such as heart failure, venous insufficiency, and renal diseases, can lead to fluid accumulation. During the day, fluid may accumulate in the lower body, such as the legs. While sleeping in a horizontal position during the night, the fluid shifts from the lower body to the upper body and influences the upper airway. A diuretic shifts fluid, e.g., water, from the body. The fluid is then removed from the body as urine. The amount of fluid shift has an influence on the amount of fluid remaining at the upper airway. By taking into account the effect of the diuretic, the setting of the therapy device is determined. In case the diuretic has not yet shifted a large amount of fluid, the setting is, for example, set to a high intensity of the therapy. In case the diuretic has shifted a large amount of fluid, the setting is, for example, set to a low intensity of the therapy. In case the diuretic has worn off, fluid may accumulate again at the upper airway due to the fluid shift by remaining fluid from the lower body to the upper body. The diuretic may not be able to remove all excess fluid from the body. As a result, the pharyngeal collapsibility may become higher during the sleep session after the diuretic has worn off. As a result, the setting is determined to provide the SDB therapy accordingly.

For example, the diuretic requires a certain amount of time to complete the fluid shift. This time may be short, such as less than 1 hour, or may be longer, such as to about 4 hours for some thiazides. The first time period is after the diuretic has completed the fluid shift. After completion of the fluid shift, the diuretic has the largest influence on the pharyngeal collapsibility, as the pharyngeal collapsibility is improved the most. The second time period is before the diuretic has completed the fluid shift. During the second time period, the diuretic may provide some improvement of the pharyngeal collapsibility. For example, during the first time period, the setting of the therapy device is set to a low intensity of the therapy. During the second time period, the setting is set to a high intensity of the therapy.

In an embodiment, the method comprises estimating a urinary time period in the sleep session based on the medication information. During the urinary time period the subject has likely a need to urinate. The method comprises determining the setting of the therapy device to provide the SDB therapy at a lower intensity level during the urinary time period than before the urinary time period.

The diuretic shifts the fluid from the body. As a result, the shifted fluid is collected in the bladder of the subject. Typically, a diuretic causes the subject to need to urinate during the sleep session. The need to urinate causes arousals that eventually wake up the subject, so the subject can go to the toilet. The method estimates when the subject is likely to wake up to go to the toilet, i.e., when the urinary time period is. During the urinary time period the setting of the therapy device is set to a lower intensity level than before. Because of the lower intensity level, the subject wakes up while receiving the SDB therapy at the lower intensity level. This causes less discomfort while waking up. Because of this less discomfort, the subject experiences less stress while waking up. Because of the less stress, the subject is more likely to fall asleep quickly after returning from the toilet, and is more likely to continue with the SDB therapy for the rest of the sleep session. For example, the urinary time period is based on medication information, such as the half-life and/or the dosage and/or the PK/PD model of the diuretic. For example, the urinary time period is based on when the subject needed to urinate during previous sleep sessions. This is based, for example, on input from the subject via a user interface. The user interface displays, for example, questions about when the subject needed to urinate. For example, the therapy device registers when the subject interrupts the SDB therapy during the sleep session. For example, the subject switches off the therapy device, or removes a patient interface of the respiratory support device, or removes the MAD from the mouth, or removes the positional therapy device from the body. Such an interruption may be considered to be caused by the subject going to the toilet, especially when the interruption is a specific duration. The specific duration is about the time the subject needs to be to the toilet and resume the therapy, which is, for example, a few minutes, such as less than 10 minutes or less than 5 minutes. The therapy device provides an interruption signal representative of the subject interrupting the SDB therapy during the sleep session. The urinary time period is based on the interruption signal.

In an embodiment, the method comprises receiving blood pressure information representative of a blood pressure of the subject, and determining the trait information based on the blood pressure information.

Because diuretics remove water from the body, diuretics have an influence on the blood pressure of the subject. For example, the diuretic reduces the blood pressure. Based on this insight, blood pressure information is used to determine the trait information. For example, a lower blood pressure or a decreasing blood pressure is indicative that the diuretic is actively shifting fluid from the body. As a result, the pharyngal collapsibility is decreasing. For example, a higher blood pressure or an increasing blood pressure is indicative that the diuretic has worn off and that the pharyngal collapsibility is increasing.

In an embodiment, the medication comprises a weight loss medication. The method comprises providing a weight loss estimation based on the medication information, and determining the trait information based on the weight loss estimation.

The use of weight loss medication may cause a faster loss of weight than dieting without the use of weight loss medication. Body weight has an important effect on the pharyngal collapsibility due to the adipose tissue in the neck and oral region. By making use of the information relating to the weight loss medication, the setting of the therapy device is adjusted. For example, when the subject has just started using weight loss medication, the subject still has a large body weight. This is the second time period, in which the weight loss medication has a small influence on the pharyngal collapsibility. During the second time period, the therapy device is set, for example, to provide the SDB therapy at a high intensity level. After the subject has been using the weight loss medication for a prolonged time, the subject has a reduced body weight. This is the first time period, in which the weight loss medication has a large influence on the pharyngal collapsibility. During the first time period, the therapy device is set, for example, to provide the SDB therapy at a lower intensity level. For example, the medication information comprises information about a weight loss over time as a result of the weight loss medication. For example, this information is based on an average weight loss over time of a group of subjects taking the weight loss medication. For example, the weight loss medication comprises a GLP1-agonist, an STGLT2-inhibtor, or a lipases inhibitor, or liraglutide, or semaglutide, or naltrexone and bupropion, or orlistat.

In an embodiment, the method comprises receiving neck circumference information or body weight information representative of a body weight of the subject, and determining the trait information based on the neck circumference information or the body weight information.

The neck circumference is indicative of the amount of adipose tissue in the neck region. The amount of adipose tissue in the neck region has a large effect on the pharyngal collapsibility. There is a relationship between body weight and neck circumference, such as indicated in the paper by Moura BAB, et.al. "Neck and waist circumference values according to sex, age, and body-mass index: Brazilian Longitudinal Study of Adult Health" (ELSA-Brasil). Braz J Med Biol Res. 2020;53(10):e9815. doi: 10.1590/1414-431x20209815. Epub 2020 Aug 17. PMID: 32813851; PMCID: PMC7433850. By making use of the neck circumference information and/or the body weight information, the trait information can be determined more accurately. For example, the neck circumference information or the body weight information is received from a medical record or via a user interface. The user interface requests the subject to provide the neck circumference information and/or the body weight information. The subject is, for example, able to measure the neck circumference using a measuring tape. For example, the subject is able to take a picture of the neck. The neck circumference information is based on the picture. For example, the body weight information is provided by an electronic scale. The method comprises receiving a body weight signal from the electronic scale, wherein the body weight signal is representative of the body weight of the subject.

In an embodiment, the method comprises receiving AHI information representative of an Apnea Hypopnea Index (AHI) of the subject during a sleep session without SDB therapy. The method comprises determining the setting of the therapy device based on the AHI information.

AHI is the apnea/hypopnea index, which represents the number of apnea events and hypopnea events per hour of sleep. AHI is a well-known measure for the severity of OSA. For a subject with an excessive body and a high pharyngal collapsibility, but with at most only a small contribution of any of the other three pathophysiological traits, a correlation between the weight loss and the AHI relates to the severity of the OSA. The paper by Locke, B.W., et al., 2024, "The association of weight loss from anti-obesity medications or bariatric surgery and apnea-hypopnea index in obstructive sleep apnea", Obesity Reviews, pp. 1-11. https://doi.org/10.1111/obr.13697, shows, for example, that the AHI is reduced with 0.45 events per hour for every 1% of body weight loss. So by taking the AHI information and at least one of the weight loss estimation, the neck circumference information, or the body weight information into account in the trait information, the setting of the therapy device is set to provide improved SDB therapy. For example, in case the weight loss estimation, neck circumference information, and/or the body weight information indicates that the subject has lost weight, but the AHI information indicates that the AHI has not decreased, the subject may have one of the other pathophysiological traits in addition to a high pharyngal collapsibility. The setting of the therapy device is adjusted to provide SDB therapy to treat the other pathophysiological trait.

In an embodiment, the medication comprises a nasal decongestant.

A nasal decongestant is a medication that relieves nasal congestion in the upper airway. By reducing or removing any nasal congestion, nasal breathing of the subject is improved. The nasal breathing helps to reduce the likelihood of collapse of the pharyngal. For example, the nasal decongestant comprises pseudoephedrine or phenylephrine or oxymetazoline.

### HIGH LOOP GAIN

According to a third aspect of the invention, there is provided a method according to the first aspect or the second aspect, wherein the pathophysiological trait comprises a high loop gain.

Loop gain is largely dominated by a ventilatory response to changes in arterial blood gases, and the interaction between a ventilatory drive and muscle compensation required for upper airway patency. An increase in ventilatory drive activates the upper airway muscles, and promotes airway patency. Likewise, a decrease in ventilatory drive relaxes the upper airway muscles, and facilitates closure and consequently, obstructions of the upper airway. Ventilatory drive can be decomposed into a chemical drive and a non-chemical drive. The chemical drive is caused by changes in arterial blood gases, such as changes in CO2 and 02. An increase in CO2 and/or a decrease of O2 increases the chemical drive. The non-chemical drive is a wakefulness response to disturbances such as arousals.

When the loop gain is too high, an overshoot in ventilatory drive occurs in response to the changes in the arterial blood gases. The overshoot causes hyperpnea, for example during or immediately following an arousal. The hyperpnea decreases the chemical drive. The decrease of the chemical drive causes a reduction of the activation of the upper airway muscles by the ventilatory drive. Due to the reduced activation of the muscles, the upper airway becomes obstructed. Because of the obstruction, the arterial blood gases change and cause another overshoot in ventilatory drive. So the high loop gain triggers subsequent obstructions, arousals and corresponding ventilatory drive overshoots, leading to the recognizable "trains of apneas", i.e., a series of consecutive SDB events.

As a result, the high loop gain has an influence on the SDB of the subject. The subject uses one or more medications that influence the high loop gain. For example, the medication comprises a medication that improves the CO2 sensing of the subject or that improves the ventilatory control of the subject. For example, the medication comprises a carbonic anhydrase inhibitor. For example, the medication comprises a medication to reduce the high loop gain, such as topiramate/phentermine, acetazolamide, sulthiame, or leptin.

As the medication has an influence on the high loop gain, the medication influences the SDB. By making use of the trait information about the high loop gain, the setting of the therapy device is improved. With the improved setting of the therapy device, the therapy device provides improved SDB therapy to the subject.

In an embodiment, the invention according to the third aspect is combined with any of the embodiments according to the first aspect of the invention or the second aspect of the invention.

In an embodiment, the therapy device is adapted to provide oxygen to the subject. Determining the setting of the therapy device comprises determining a flow rate of the oxygen to the subject based on the trait information.

Oxygen is known to stabilize ventilation, see for example the paper of Wellman, et.al. "Effect of Oxygen in Obstructive Sleep Apnea: Role of Loop Gain." Respiratory Physiology & Neurobiology 162, no. 2 (July 2008): 144-51. https://doi.org/10.1016/j.resp.2008.05.019. So by providing additional oxygen to the subject while providing the SDB therapy, the high loop gain is reduced. For example, during the first time period while the medication has a large influence on the high loop gain, only a small amount or no additional oxygen is provided to the subject. During the second time period, when the medication has only a small or no influence on the high loop gain, a sufficient amount of additional oxygen is provided to the subject. For example, the setting sets the oxygen concentration in the pressurized air provided by the respiratory support device. For example, oxygen is provided to the subject in addition to the subject using the positional therapy device or using the MAD. For example, the therapy device comprises a patient interface to provide the oxygen to the subject, such as full face mask or an orinasal mask, or a nasal mask, or a nasal pillow. For example, the patient interface is adapted to connect to cannister. The cannister holds a supply of oxygen. For example, the therapy device is adapted to mix the oxygen from the cannister with ambient air, before providing the oxygen to the subject.

In an embodiment, the therapy device is adapted to provide carbon dioxide to the subject. Determining the setting of the therapy device comprises determining a flow rate of the carbon dioxide to the subject based on the trait information.

According to this embodiment, carbon dioxide is provided to the subject. The carbon dioxide raises the baseline of the chemical drive. The raised baseline of the chemical drive helps to increase the minimum activation of the upper airway muscles. As a result of the increased minimum activation of the upper airway muscles, the airway is obstructed less. For example, during the first time period while the medication has a large influence on the high loop gain, only a small amount or no additional carbon dioxide is provided to the subject. During the second time period, when the medication has only a small or no influence on the high loop gain, a sufficient amount of carbon dioxide is provided to the subject. For example, the setting sets the carbon dioxide concentration in the pressurized air provided by the respiratory support device. For example, carbon dioxide is provided to the subject in addition to the subject using the positional therapy device or using the MAD. For example, the therapy device comprises a patient interface to provide the carbon dioxide to the subject, such as full face mask or an orinasal mask, or a nasal mask, or a nasal pillow. For example, the patient interface is adapted to connect to cannister. The cannister holds a supply of carbon dioxide. For example, the therapy device is adapted to mix the carbon dioxide from the cannister with ambient air, before providing the carbon dioxide to the subject. The concentration of carbon dioxide provided to the subject is preferably higher than the concentration of carbon dioxide of ambient air to raise the baseline of the chemical drive, but is low enough to prevent symptoms relating to carbon dioxide poisoning, such as headache or dizziness or rapid breathing.

In an embodiment, the method comprises receiving respiratory information representative of respiration of the subject during a sleep session. The method comprises determining a beginning of an airflow limitation based on the respiratory information. The method comprises determining whether the airflow limitation is still occurring after a maximum waiting time. The method comprises, in case the airflow limitation is still occurring after the maximum waiting time, generating a stimulation signal. The stimulation signal is adapted to cause a stimulator to provide a stimulus to the subject. The stimulus is adapted to cause arousal of the subject.

According to this embodiment, the beginning of an airflow limitation is determined based on the respiratory information. Because of the airflow limitation, the arterial blood gases change, causing an increase in the chemical drive. In case the airflow limitation still occurs after a maximum waiting time, the chemical drive has increased to a certain level. In case the chemical drive would increase even more, the chemical drive would cause an arousal. In response to the arousal, the non-chemical drive would be activated. The combined non-chemical drive and the high chemical drive would cause an overshoot in ventilation. However, the stimulation signal is generated at the maximum waiting time. The stimulation signal causes the stimulator to provide the stimulus. The stimulus causes an arousal of the subject, and activates the non-chemical drive. In this case, the combination of the non-chemical drive and the smaller chemical drive does not cause an overshoot in ventilation. As a result, the ventilation does not become instable and does not create a series of SDB events. For example, the stimulator is a light source adapted to generate a light signal, such as a light flash or a series of light flashes. For example, the stimulator is sound generator adapted to generate a sound, such as a beep or noise or music. For example, the stimulator is a vibrator adapted to generate a vibration or a series of vibrations.

In an embodiment, the method comprises receiving information about a eupneic ventilation of the subject. The method comprises receiving respiratory information representative of respiration of the subject. The method comprises determining the maximum waiting time by: detecting a plurality of airflow limitations based on the respiratory information; waiting a waiting time after a start of each airflow limitation, wherein the waiting time is different for each airflow limitation; generating a stimulation signal when the waiting time expires, wherein the stimulation signal is adapted to cause a stimulator to provide a stimulus to the subject; receiving ventilation information representative of a ventilation of the subject after the stimulus; comparing the ventilation information with the eupneic ventilation; and selecting as the maximum waiting time the waiting time corresponding to the ventilation of the subject being different from the eupneic ventilation within a threshold.

The maximum waiting time may be different for different subjects. The maximum waiting time of a subject may vary over time. Therefore, it is beneficial to determine the maximum waiting time based on the respiratory information of the subject. The method receives information about the eupneic ventilation to have a reference value of a healthy ventilation of the subject. For example, the eupneic ventilation is based on a medical record of the subject, or a measurement of the subject when the subject is breathing without airflow limitation. For example, the eupneic ventilation is based on ventilation while the subject is awake. The respiratory information includes, for example, information about ventilation and/or airflow and/or respiration effort of the subject. A plurality of airflow limitations is detected based on the respiratory information. For example, when a first airflow limitation occurs, the stimulation signal is generated in case the first airflow limitation still occurs after a first waiting time expires. The stimulator provides the stimulus to the subject based on the stimulation signal. The ventilation information indicates any changes in ventilation in response to the stimulus. For example, the ventilation information indicates a stabile ventilation or an instable ventilation in response to the stimulus. The ventilation information is compared to the eupneic ventilation. In case the comparison indicates that the ventilation in response to the stimulus matches well with the eupneic ventilation, the waiting time is set as the maximum waiting time. Alternatively, the steps to determine the maximum waiting time are repeated for the next airflow limitation using a longer waiting time to see whether the longer waiting time also results in a ventilation in response to the stimulus that matches well with the eupneic ventilation. In case the comparison indicates that the ventilation in response to the stimulus does not match well with the eupneic ventilation, the steps to determine the maximum waiting time are repeated for the next airflow limitation using a shorter waiting time to see whether the shorter waiting time results in a ventilation in response to the stimulus that matches well with the eupneic ventilation. The shorter the waiting time, the less time the chemical drive is able to increase. The lower the chemical drive, the less risk for an overshoot in ventilation. The threshold indicates whether the ventilation matches well with the eupneic ventilation.

In an embodiment, the therapy device comprises a respiratory support device adapted to provide pressurized air to the subject. The therapy device comprises the stimulator. The stimulation signal is adapted to cause the respiratory support device to provide the pressurized air to the subject in a series of one or more pulses.

According to this embodiment, the respiratory support device provides as the stimulus a series of pressurized air pulses in response to the stimulation signal. The series of one or more pulses cause an arousal of the subject. This embodiment allows the respiratory support device to provide the stimulus without the need of any additional component for generating the stimulus.

In an embodiment, the therapy device comprises a respiratory support device adapted to provide pressurized air to the subject. The method comprises determining the trait information during a first time period and during a second time period. During the first time period the influence of the medication on the high loop gain is larger than during the second time period. The method comprises determining the setting of the therapy device to provide the pressurized air with an inspiration pressure and an expiration pressure. A difference between the inspiration pressure and the expiration pressure is larger during the second time period than during the first time period.

During both the first time period and the second time period, the expiration pressure is large enough to help prevent the upper airway from collapsing during exhalation. However, during the second time period, the chemical drive is more likely to become too small to sufficiently activate the upper airway muscles to timely inhale. By having a larger difference between the inspiration pressure and the expiration pressure during the second time period than during the first time period, the inspiration pressure during the second time period helps to timely inhale and thus helps to prevent SDB events. This larger inspiration pressure is not needed during the first time period, as the chemical drive is sufficient due to the influence of the medication. For example, the expiration pressure is the same in the first time period and the second time period. For example, the inspiration pressure is the lower in the first time period than in the second time period.

In an embodiment, the therapy device comprises a respiratory support device adapted to provide pressurized air to the subject. The method comprises receiving respiratory information representative of respiration of the subject during a sleep session. The method comprises determining an SDB event based on the respiratory information. The method comprises determining the setting of the therapy device to provide the pressurized air with an inspiration pressure and an expiration pressure. A difference between the inspiration pressure and the expiration pressure is larger after the SDB event than before the SDB event.

According to this embodiment, the SDB event may cause an overshoot in ventilation and may cause a series of SDB events due to the high loop gain. By increasing the difference between the inspiration pressure and the expiration pressure after the SDB event, the stability of the ventilation is improved and the series of SDB events may be prevented. For example, the increased difference is provided for less than 10 breathing cycles after the SDB event, such as for 3 or 5 breathing cycles. When there is no SDB event, such as before the SDB event, the difference between the inspiration pressure and the expiration pressure is smaller. For example, before the SDB event the inspiration pressure and the expiration pressure are equal to each other.

In an embodiment, the medication comprises a carbonic anhydrase enzyme inhibitor (CAI).

Carbonic anhydrase inhibitors are a class of pharmaceuticals that suppress the activity of carbonic anhydrase. CAI's are used to treat glaucoma, epilepsy, and mountain sickness. Recent studies show that CAI's are beneficial in treating OSA as CAI's influence high loop gain. An example of a CAI is acetazolamide.

In an embodiment, the method comprises receiving oxygen saturation information representative of an oxygen saturation of the subject, and determining the trait information based on the oxygen saturation information.

A study by Leaf, D.E. and Goldfarb, D.S., 2007, "Mechanisms of action of acetazolamide in the prophylaxis and treatment of acute mountain sickness", J Appl Physiol 102: 1313-1322, https://doi.org/10.1152/japplphysiol.01572.2005 about mountain sickness, shows that CAI's, such as acetazolamide, have an influence on the oxygen saturation. So by using the oxygen saturation information, information is obtained about the influence of the CAI on the body of the subject. For example, the oxygen saturation information is generated by an oxygen saturation sensor, such as a photoplethysmographic (PPG) sensor coupled to the subject. For example, the CAI is acetazolamide.

In an embodiment, the method comprises receiving respiratory information representative of respiration of the subject. The method comprises determining a presence of a plurality of SDB events based on the respiratory information. The method comprises determining, for each of the plurality of SDB events, a ventilation overshoot, a ventilation undershoot or a ventilation time period between the ventilation overshoot and the ventilation undershoot. The method comprises determining a trend based on the ventilation overshoot, the ventilation undershoot or the ventilation time period for each of the plurality of SDB events. The method comprises determining the trait information based on the trend.

The ventilation overshoot, the ventilation undershoot, and the ventilation time period depend on the severity of the high loop gain. By looking at the trend, the influence of the medication on the high loop gain can be determined. In case the trend shows an improvement of the high loop gain, such as fewer and less severe ventilation overshoots and ventilations undershoots, and longer ventilation time periods, the medication is increasing the influence on the high loop gain. In case the trend shows a worsening of the high loop gain, such as more and more severe ventilation overshoots and ventilations undershoots, and shorter ventilation time periods, the medication is decreasing the influence on the high loop gain. The trait information is based on the trend.

In an embodiment, the method comprises receiving sleep stage information representative of Rapid Eye Movement (REM) sleep and non-REM sleep during sleep of the subject. The method comprises determining a change in a difference between a first loop gain metric and a second loop gain metric during the sleep of the subject. The first loop gain metric is representative of an amount of loop gain during the REM sleep. The second loop gain metric is representative of an amount of loop gain during the non-REM sleep. The method comprises determining the trait information based on the change in the difference.

A study by Messineo, L. et al., 2019, "Loop gain in REM versus non-REM sleep using CPAP manipulation: A pilot study", Respirology (2019) 24, 805-808 https://doi.org/10.1111/resp.13G08, shows that the loop gain during REM sleep is about 25% lower than the loop during non-REM sleep. Determining REM sleep and non-REM sleep is known based on, for example, cardiac properties and/or respiratory properties of the subject. A sleep staging system to determine REM sleep and non-REM sleep is described in US stated patent US 11,484,256 B2, hereby incorporated by reference. The first loop gain metric and the second loop gain metric are, for example, based on the amount of SDB events, the number and/or severity of ventilation overshoot, the number and/or severity of ventilation undershoot, and/or the ventilation time period. In case the medication to reduce the high loop gain has a large influence, the loop gain is reduced. As a result, the about 25% difference between REM sleep and non-REM sleep is smaller than in the case the medication has a small influence or no influence. In case the medication has a small influence or no influence, the loop gain is high. 25% Of the high value results in a larger difference between REM sleep and non-REM sleep. As a result, the change in the difference is indicative of the influence of the medication on the high loop gain.

In an embodiment, the method comprises receiving respiratory information representative of respiration of the subject. The method comprises determining a presence of a plurality of SDB events based on the respiratory information. The method comprises determining an inter-event time between two consecutive SDB events of the plurality of SDB events. The method comprises determining the trait information based on the inter-event time.

A characteristic of a high loop gain is a so called "train" of SDB events. The ventilation overshoot and the subsequent ventilation undershoot after a SDB event triggers a next SDB event. Therefore, the spacing between the SDB events, i.e., the inter-event time, is representative of the loop gain. When the inter-event time is long, the medication sufficiently reduces the loop gain. In case the inter-event is short or becomes shorter, the influence of the medication is reduced or reducing. The trait information is based, for example, on multiple inter-event times, or a trend of the inter-event times. The trend of the inter-event times indicates, for example, that the inter-events times are increasing, or are decreasing, or remain stable. For example, the inter-event times indicate a series of SDB events with short inter-events times in between. The last SDB event in the series is followed by a longer inter-event time. For example, the short inter-events times are the duration of one or two breathing cycles. For example, the longer inter-event time is at least three breathing cycles or at least 10 breathing cycles.

### LOW MUSCLE RESPONSIVENESS

According to a fourth aspect of the invention, there is provided a method according to any one of the first aspect, the second aspect, or the third aspect, wherein the pathophysiological trait comprises a low muscle responsiveness of muscles in an upper airway of the subject.

In healthy subjects, the ventilatory drive activates the muscles of the upper airway to keep the airway open during inhalation and exhalation. The response of the muscles to the ventilatory drive should be high enough to ensure the airway is open during inhalation and exhalation. However, the subject has a pathophysiological trait that comprises a low muscle responsiveness. For example, the muscles receive only a weak neurological signal in response to the ventilatory drive. The weak neurological signal does not sufficiently activate the muscles. For example, the muscles are weak and cannot contract enough in response to ventilatory drive to open the airway. In both examples, the muscles have a low responsiveness to the ventilatory drive. The upper airway may be partly or completely obstructed because the low responsiveness of the muscles causes insufficient activation of the muscles to open the upper airway.

As a result, the low muscle responsiveness has an influence on the SDB of the subject. The subject uses one or more medications that influence the low muscle responsiveness. For example, the medication comprises a medication that increases the muscle tone of the muscles in the upper airway. For example, the medication increases the muscle response to neurological signals caused by the ventilatory drive. For example, the medication comprises a noradrenergic reuptake inhibitor, or a muscarinic receptor antagonist. For example, the medication comprises a sensor response enhancer, such as a nasal application of a TASK-1-3 K channel blocker. For example, the medication comprises atomoxetine and oxybutynin. For example, the medication comprises atomoxetine and aroxybutynin. For example, the medication comprises reboxetine and oxybutynin. For example, the medication comprises reboxetine and hyoscine butylbromide.

As the medication has an influence on the low muscle responsiveness, the medication influences the SDB. By making use of the medication information and the trait information about the low muscle responsiveness, the setting of the therapy device is improved. With the improved setting of the therapy device, the therapy device provides improved SDB therapy to the subject.

In an embodiment, the invention according to the fourth aspect is combined with any of the embodiments according to the first aspect, the second aspect, or the third aspect of the invention.

In an embodiment, the method comprises receiving respiratory information representative of respiration of the subject. The method comprises determining an occurrence of an airflow limitation based on the respiratory information. The method comprises determining a baseline ventilation before the airflow limitation based on the respiratory information. The method comprises determining a recovery ventilation after the airflow limitation based on the respiratory information. The method comprises determining the influence of the medication on the low muscle responsiveness based on a difference between the baseline ventilation and the recovery ventilation.

The subject may suffer an airflow limitation, such as a partial or complete obstruction of the airway. The airflow limitation is a reduction in ventilation compared to the base line ventilation before the airflow limitation. The base line ventilation is, for example, the ventilation of the subject during sleep without airflow limitation. For example, the base line ventilation is eupneic ventilation, i.e., healthy ventilation. When the subject recovers from the airflow limitation, the ventilation increases. In case of sufficient muscle responsiveness, the ventilation returns to the baseline ventilation. In case of low muscle responsiveness, the ventilation increases after the airflow limitation, but remains at a level below the baseline ventilation. The lower the muscle responsiveness, the lower the level of ventilation during the airflow limitation. So by determining the recovery ventilation after the beginning of the airflow limitation, it can be determined how much influence the medication has on the muscle responsiveness.

In case of sufficient muscle responsiveness to an airflow limitation, the airflow limitation is not an SDB event or does not lead to an SDB event. In that case, the airflow limitation is, for example, only a small limitation and/or has only a short duration. In case of insufficient muscle responsiveness to an airflow limitation, the airflow limitation is an SDB event or leads to an SDB event. In that case, the airflow limitation is, for example, a large limitation and/or has a long duration. For example, in case the airflow limitation causes the oxygen saturation level of the subject to decrease below a threshold, and/or in case the airflow limitation leads to an arousal, the airflow limitation is a SDB event.

Ventilation is based on a tidal volume and a breathing rate. The tidal volume is the volume of air per breath. The breathing rate is the number of breaths per minute. Ventilation is expressed in volume per unit of time, such as liters per minute. A change in ventilation can be determined by determining a change in tidal volume, a change in breathing rate, or a change in the combination of tidal volume and breathing rate.

In an embodiment, the method comprises receiving electromyography (EMG) information representative of electrical activity of the muscles in the upper airway. The method comprises determining the influence of the medication on the low muscle responsiveness based on the EMG information.

EMG information is obtainable via an electrode, or a pair of electrodes, arranged at the throat area. The electrodes sense electrical signals generated by the muscles in the throat area. The muscles in the throat area are some of the muscles of the upper airway. The electrical activity is a measure of the activation or the muscle tone of the muscles. A large electrical activity represents a high muscle tone, which relates to a sufficient muscle responsiveness. A small electrical activity represents a low muscle tone, which relates to a low muscle responsiveness. So based on the EMG information, it can be determined how much influence the medication has on the muscle responsiveness.

In an embodiment, the medication comprises a norepinephrine reuptake inhibitor (NRI). The method comprises wherein the method comprises receiving heart rate information and receiving blood pressure information. The method comprises determining the influence of the medication on the low muscle responsiveness based on the heart rate information and the blood pressure information.

The medication has a component that comprises an NRI, such as atomoxetine, reboxetine, or viloxazine. Studies indicate that NRIs help to improve muscle responsiveness to treat SDB. Besides the influence on the muscle responsiveness, NRIs affect heart rate and blood pressure. For example, atomoxetine increases heart rate and blood pressure. The heart rate information and/or the blood pressure information is, for example, obtained via a PPG measurement, a pulse transit time (PPT) measurement, a blood pressure cuff, or any combination thereof. For example, the information indicates an absolute heart rate or an absolute blood pressure, or the information indicates a change in heart rate or a change in blood pressure. For example, the heart rate information is representative of a trend of the heart rate. For example, the blood pressure information is representative of a trend of the blood pressure. For example, only one of the heart rate information and the blood pressure information is used by the method. In case the information indicates an increase of heart rate and an increase of blood pressure, the effect of the NRI increases. As a result, the influence of the medication on the SDB increases. In case the information indicates a decrease of heart rate and a decrease of blood pressure, the effect of the NRI decreases. As a result, the influence of the medication on the SDB decreases. So based on the heart rate information and the blood pressure information, it can be determined how much influence the medication has on the muscle responsiveness.

In an embodiment, the medication comprises atomoxetine. The method comprises receiving heart rate information of the subject. The method comprises receiving arousal information of the subject. The method comprises determining a response of the heart rate of the subject to an arousal based on the heart rate information and the arousal information. The method comprises determining the influence of the medication on the low muscle responsiveness based on the heart rate in response to the arousal.

The medication has a component that comprises an NRI, such as atomoxetine, reboxetine, or viloxazine. According to a study by Liang, E.F., et al., "The Effect of Methylphenidate and Atomoxetine on Heart Rate and Systolic Blood Pressure in Young People and Adults with Attention-Deficit Hyperactivity Disorder (ADHD): Systematic Review, Meta-Analysis, and Meta-Regression", Int. J. Environ. Res. Public Health 2018, 15, 1789; doi: 10.3390/ijerph15081789, atomoxetine has an impact on the sympathetic nervous system. The method makes use of this impact by making use of the response of the heart rate to an arousal. In case the NRI has a large influence on the subject, the arousal causes a large increase of the heart rate. In case the NRI has a small influence on the subject, the arousal causes a small increase of the heart rate. So based on the heart rate in response to the arousal, it can be determined how much influence the medication has on the muscle responsiveness. For example, the arousal information is based on the heart rate information, as the occurrence of an arousal can be determined based on heart rate. For example, the arousal information is based on EEG information, or respiratory information.

In an embodiment, the medication comprises oxybutynin. The method comprises receiving electrical skin conductivity information. The method comprises determining the influence of the medication on the low muscle responsiveness based on the electrical skin conductivity information.

Studies indicate that oxybutynin helps to improve muscle responsiveness to treat SDB. Oxybutynin is known to treat excessive sweating, i.e., hyperhidrosis. So when the oxybutynin has a large influence on the subject, the subject sweats less than normal. The electrical skin conductivity provides a measure for the amount of sweating by the subject. So when determining an increase of sweating during a sleep session, the influence of the oxybutynin decreases. So based on the electrical skin conductivity information, it can be determined how much influence the medication has on the muscle responsiveness. For example, the electrical skin conductivity information is provided by a pair of electrodes attached to the subject. For example, the pair of electrodes is attached to a hand or a foot of the subject.

In an embodiment, the medication comprises oxybutynin. The method comprises receiving body temperature information. The method comprises determining the influence of the medication on the low muscle responsiveness based on the body temperature information.

Oxybutynin is known to increase body temperature. So when the oxybutynin has a large influence on the subject, the subject has a higher body temperature than normal. The body temperature information provides a measure for the body temperature of the subject. So when determining a decrease of the body temperature during a sleep session, the influence of the oxybutynin decreases. So based on the body temperature information, it can be determined how much influence the medication has on the muscle responsiveness. For example, the body temperature information is provided by a body temperature sensor attached to the subject.

### LOW AROUSAL THRESHOLD

According to a fifth aspect of the invention, there is provided a method according to any one of the first aspect, the second aspect, the third aspect, or the fourth aspect, wherein the pathophysiological trait comprises a low arousal threshold.

Breathing is controlled by the ventilatory drive. The ventilatory drive can be decomposed into a chemical drive and a non-chemical drive. The chemical drive is caused by changes in arterial blood gases, such as changes in CO2 and 02. An increase in CO2 and/or a decrease of O2 increases the chemical drive. The non-chemical drive is a wakefulness response, such as an arousal, to disturbances. For healthy subjects, the chemical drive can reach a certain level before the healthy subject has an arousal during sleep. When breathing of the healthy subject is obstructed during sleep, the ventilatory drive increases to a level that causes the muscles of the upper airway to overcome the obstruction. In case the chemical drive overcomes the restriction at a level below the arousal threshold, the healthy subject is able to continue sleeping during the restriction.

However, the subject has a pathophysiological trait that comprises a low arousal threshold. When breathing of the subject is obstructed during sleep, the chemical drive increases towards a level that causes the muscles of the upper airway to overcome the obstruction. However, that level of chemical drive is above the arousal threshold of the subject. So before the chemical drive is able to reach the level to overcome the obstruction, the subject has an arousal. The arousal causes the non-chemical drive of the ventilatory drive to activate the muscles of the upper airway to remove the obstruction. The arousal is associated with the wakefulness response. Because of the low arousal threshold, even minor obstructions and short obstructions of the upper airway cause arousals. The arousal causes an interruption of sleep, such as awakenings and sleep fragmentation. As a result, even minor obstructions and short obstructions of the airway cause SDB events.

As a result, the low arousal threshold has an influence on the SDB of the subject. The subject uses one or more medications that influence the low arousal threshold. For example, the medication comprises a medication that increases the low arousal threshold. For example, the medication comprises a hypnotic, such as an adrenergic blockade, or a GABA enhancer. For example, the medication comprises a sedative. For example, the medication comprises trazodone, eszopiclone, or zolpidem.

As the medication has an influence on the low arousal threshold, the medication influences the SDB. By making use of the trait information about the low arousal threshold, the setting of the therapy device is improved. With the improved setting of the therapy device, the therapy device provides improved SDB therapy to the subject.

In an embodiment, the invention according to the fifth aspect is combined with any of the embodiments according to the first aspect, the second aspect, the third aspect, or the fourth aspect of the invention.

In an embodiment, the medication comprises a hypnotic or a sedative. Both hypnotics and sedatives help to raise the arousal threshold. Hypnotics are used to treat insomnia and induce sleep. Sedatives help to relax the subject and to remove anxiety. For example, the subject is prescribed with a sedative for a condition other than SDB, such as a panic disorder or anxiety or pain relief. As an additional effect, the sedative helps to improve the arousal threshold.

In an embodiment, the method comprises determining an influence of the medication on the low arousal threshold for a first time period and a second time period. During the first time period the influence of the medication on the low arousal threshold is larger than during the second time period. The method comprises determining the setting to provide the SDB therapy at a higher intensity level during the first time period than during the second time period.

In an embodiment, the method comprises determining an influence of the medication on the low arousal threshold for a first time period and a second time period. During the first time period the influence of the medication on the low arousal threshold is larger than during the second time period. The method comprises determining whether the subject is in a supine position. The method comprises generating a stimulation signal when the subject is in the supine position. The stimulation signal is adapted to cause a stimulator to provide a stimulus to the subject to prompt the subject to change to a body position different than the supine position. The stimulus is provided at a higher intensity level during the first time period than during the second time period.

According to this embodiment, the SDB is treated by stimulating the subject to move out of the supine position, as the supine position worsens the SDB. During the first time period, the medication increases the arousal threshold more than during the second time period. As a result, in the first time period, the subject requires more intense stimulation to arouse the subject to move out of the supine position. However, the high intensity of the first time period may cause the subject to wake up during the second time period. During the second time period, a lower intensity stimulation is provided, which is sufficient because of the lower arousal threshold.

In an embodiment, the method comprises receiving sleep architecture information representative of a first sleep architecture and a second sleep architecture of the subject. The first sleep architecture is during a first time period. The second sleep architecture is during a second time period. The method comprises determining a change between the first sleep architecture and the second sleep architecture based on the sleep architecture information. The method comprises determining the influence of the medication on the low arousal threshold based on the change between the first sleep architecture and the second sleep architecture.

Sleep architecture is affected by the arousal threshold. In case of a low arousal threshold, the sleep architecture shows, for example, more sleep fragmentation, more sleep stage transitions, and/or more awakenings compared to the case with a higher arousal threshold. So based on the sleep architecture information, it can be determined how much influence the medication has on the arousal threshold. For example, a baseline of the sleep architecture is the sleep architecture of the subject while not taking the medication. For example, the sleep architecture is determined based on sleep stages of the subject detected by a neurologic parameter, such as EEG, and/or a cardiac parameter, such as heart rate or heart rate variability, and/or a respiratory parameter, such as breathing rate or breathing variability.

In an embodiment, the medication comprises an orexin receptor antagonist. The method comprises the change between the first sleep architecture and the second sleep architecture comprises a change in deep sleep and/or a change in REM sleep.

A study by Clark JW, et.al. "Effects of orexin receptor antagonism on human sleep architecture: A systematic review." Sleep Med Rev. 2020 Oct;53: 101332. doi: 10.1016/j.smrv.2020.101332. Epub 2020 May 13. PMID: 32505969, shows that orexin receptor antagonists affect deep sleep and REM sleep. Deep sleep and REM sleep are sleep stages that form part of the sleep architecture. So based on the change in deep sleep and/or REM sleep, it can be determined how much influence the medication has on the arousal threshold. For example, the change in deep sleep and/or REM sleep is during a sleep session. For example, the natural increase of REM sleep during a sleep session may be taken into account to determine the influence of the medication. For example, the change in deep sleep and/or REM sleep is compared to a sleep session of the subject in which the subject did not take the medication.

In an embodiment, the method comprises generating a stimulation signal. The stimulation signal is adapted to cause a stimulator to provide a stimulus to the subject. The method comprises receiving a stimulus response signal representative of a response of the subject to the stimulus. The method comprises determining the influence of the medication on the low arousal threshold based on the stimulus response signal.

The stimulus is provided in an attempt to arouse the subject. If the subject is aroused, the intensity of the stimulus is above the arousal threshold. If the subject is not aroused, the intensity of the stimulus is not above the arousal threshold. This way, information about the arousal threshold is obtained. Based on the information about the arousal threshold, the influence of the medication on the low arousal threshold can be determined. For example, the stimulation signal causes the stimulator to provide the stimulus at a certain intensity level. In case the stimulus response signal indicates that the subject is not aroused by the stimulus, a further stimulation signal is provided. The further stimulation signal causes the stimulator to provide a stimulus with a higher intensity level. The stimulation signal is generated repeatedly to causes stimuli with increasing intensity levels till the stimulus response signal indicates the subject is aroused. The intensity level corresponding to the stimulation signal that caused the arousal is a measure for the arousal threshold. For example, the arousal threshold is determined in this way, during the sleep session, every half hour or every hour or every two hours. In another example, the stimulation signal causes the stimulator to provide the stimulus at a certain intensity level. The stimulus response signal indicates the level of response to the stimulus. For example, the subject does not respond at all, the subject responds moderately, or the subject response excessively. For example, the subject responds moderately with a limited increase in heart rate. For example, the subject response excessively by waking up. For example, in case the subject does not respond to the stimulus, the medication has a high influence on the arousal threshold. For example, in case the subject has a moderate or excessive response to the stimulus, the medication has a lower influence on the arousal threshold. The stimulus response signal is, for example, based on a change in heart rate or heart rate variability, or a change in breathing rate or breathing rate variability, or movement of the subject, or a neurological parameter, such as EEG, of the subject.

In an embodiment, the stimulator comprises a light generator, a sound generator, or a vibration generator, or a pulsed air generator.

According to this embodiment, the stimulator may use light or sound or vibration or an pulse of air as a stimulus. For example, the light generator generates red or infrared light to stimulate the eyes through the closed eyelids. For example, the vibration generator is attached to the subject, such as at the chest or at the neck, for example by using a belt. The sound generator is, for example, adapted to generate a beep, or music, or nature sounds. For example, the stimulator is integrated in the therapy device or is arranged separately from the therapy device. For example, the stimulator is arranged in the patient interface of the respiratory support system. For example, the respiratory support system is adapted to provide the pulsed air to the subject via the patient interface.

In an embodiment, wherein the stimulus response signal is representative of a cardiac parameter, a neurological parameter or a respiratory parameter or a body movement parameter.

The stimulus response signal is, for example, representative of a cardiac parameter, such as heart rate, or heart rate variability, or an interbeat interval (IBI) between two successive heart beats. For example, the cardiac parameter is detected based on a PPG measurement, or an acceleration measurement, or an electrocardiographic (ECG) measurement. The stimulus response signal is, for example, representative of a neurological parameter, such brain activity, or muscle activity, eye movement activity. For example, the neurological parameter is based on an electroencephalographic (EEG) measurement, or an electromyographic (EMG) measurement, or an electrooculographic (EOG) measurement. The stimulus response signal is, for example, representative of a respiratory parameter, such as ventilation, or breathing rate, or tidal volume, or inhalation, or exhalation. For example, the respiratory parameter is detected based on a flow rate measurement, or an air pressure measurement, or a movement measurement of the chest. The stimulus response signal is, for example, a sudden change in the respiratory parameter.

In an embodiment, the method comprises receiving sleep spindle information representative of occurrences of sleep spindles of the subject. The method comprises determining the influence of the medication on the low arousal threshold based on the sleep spindle information.

Sleep spindles are short bursts of brain activity during non-REM sleep. Sleep spindles help to maintain stable sleep. For example, in case the number of sleep spindles remain stable during the sleep session, the arousal threshold is large enough. In case the number of sleep spindles reduces during the sleep session, the medication no longer has a high influence on the arousal threshold. For example, in case the number of sleep spindles remain stable while providing the stimulus based on the stimulation signal, the arousal threshold is large enough. In case the number of sleep spindles reduces in response to the stimulus, the medication no longer has a high influence on the arousal threshold.

In an embodiment, the method comprises receiving ventilatory information representative of ventilation of the subject during the sleep session. The method comprises receiving arousal information corresponding to the occurrence of at least two arousals during the sleep session. The method comprises determining a level of ventilation for each occurrence of the at least two arousals based on the ventilatory information and the arousal information. The method comprises comparing the levels of ventilation with each other. The method comprises determining the trait information based on comparing the levels of ventilation with each other.

According to this embodiment, the occurrence of arousals during the sleep session are detected, for example using EEG, or any other suitable surrogate modality, as described in other parts of this patent application. For each arousal, a corresponding level of ventilation is determined. The differences between the levels of ventilation at which the arousals occur are a measure of the arousal threshold. In case the arousals occur at increasing levels of ventilation, the arousal threshold is increasing. In case the arousals occur at decreasing levels of ventilation, the arousal threshold is decreasing. In case the arousals all occur at the same level of ventilation, the arousal threshold is not changing. Based on this information, the influence of the medication on the low arousal threshold is determined.

### CHANGES IN DOSAGE

According to a sixth aspect of the invention, there is provided a method according to any one of the first aspect, the second aspect, the third aspect, the fourth aspect, or the fifth aspect. The medication information is representative of a first dosage during a first dosage period and a second dosage during a second dosage period. The first dosage is higher than the second dosage. The method comprises determining the setting of the therapy device to provide the SDB therapy having a therapy parameter, wherein the therapy parameter has a first value during the first dosage period and has a second value during the second dosage period, wherein the first value is different from the second value.

Using a medication for a prolonged time can have adverse effects. For example, the subject may create a dependency on the medication, or in other words, may become addicted to the medication. For example, the prolonged time of using the medication has caused the subject to build a tolerance to the medication. As a result of the tolerance, the medication is less effective. For example, the subject has to undergo a certain medical treatment, such as surgery, and the medication complicates that medical treatment. Therefore, it is beneficial to reduce the medication from a first dosage to a second dosage. However, because the second dosage is lower than the first dosage, the medication has less influence on the pathophysiological trait during the second dosage period than during the first dosage period. As a result of the reduced influence, the SDB of the subject is worse during the second dosage period than during the first dosage period. The setting of the therapy device for the first dosage period is not sufficient to provide proper SDB therapy during the second dosage period. By making use of the medication information providing information about the first dosage during the first dosage period and the second dosage during the second dosage period, the setting of the therapy device to provide the SDB therapy is set to change the therapy parameter from the first value during the first dosage period to the second value during the second dosage period. As a result, the therapy device provides proper SDB therapy during the second dosage period. As a result, improved SDB therapy is provided with the therapy device for the subject using medication for treating the SDB.

In an embodiment, the medication information comprises a dosage reduction scheme during a dosage reduction period. The dosage reduction period is between the first dosage period and the second dosage period. The dosage reduction scheme is representative of a dosage reduction from the first dosage to the second dosage over the dosage reduction period. The method comprises determining the setting of the therapy device to adjust the therapy parameter during the dosage reduction period based on the dosage reduction scheme.

The dosage reduction scheme prescribes the dosage over the dosage reduction period to reduce the dosage from the first dosage to the second dosage. For example, the dosage reduction scheme helps to reduce withdrawal symptoms or side effects of the medication. During the dosage reduction period, the influence of the medication on the pathophysiological trait reduces. By determining the setting to adjust the therapy parameter, improved SDB therapy is provided during the dosage reduction period. For example, the dosage reduction period is a week, or multiple weeks, or multiple months.

In an embodiment, the method comprises receiving sleep information representative of a sleep parameter during the dosage reduction period or the second dosage period. The sleep parameter is representative of a sleep quality of the subject. The method comprises determining, based on the sleep information, whether the sleep parameter is indicative of a deterioration of the sleep quality. The method comprises, in response to determining the deterioration of the sleep quality, determining the setting of the therapy device to adjust the therapy parameter.

Because of the reduction of the dosage, the therapy device provides more intense SDB therapy to properly treat the subject. However, this may cause the sleep quality to become worse. In case the sleep quality becomes unacceptable to the subject, the subject may no longer adhere to the SDB therapy and/or may suffer from daytime sleepiness. So by determining the setting to adjust the therapy parameter, a balance is achieved between an acceptable sleep quality and proper SDB therapy. For example, the therapy parameter relates to the intensity level of the SDB therapy. In case the sleep quality deteriorates, the associated intensity level is maintained, or the intensity level is decreased. For example, in case the sleep quality deteriorates, the therapy parameter is restored to a value at which the sleep quality did not deteriorate.

In an embodiment, the sleep parameter comprises one of a user input, sleep stage information, sleep architecture information, sleep quality, or sleep time misperception.

User input is an effective measure of sleep quality and about whether the user is able adhere to the SDB therapy. The user input is, for example, an indication whether the subject perceived having slept well or not. For example, the subject rates the sleep quality with a score, such as a score of 1-5 or 1-10, where the lowest value indicates very poor sleep quality, and the highest value indicates very good sleep quality. For example, the subject rates the sleep quality with a rating on a qualitative scale ranging from very poor to very good. Sleep stage information provides information, for example, about how much time the subject slept and how much time the subject was awake, about the amount of deep sleep, the amount of REM sleep, the number of awakenings, and/or the number of sleep stage transitions. The sleep quality, or a change in sleep quality, can be determined based on this information. The sleep parameter may be sleep quality itself. For example, the sleep quality is based on a questionnaire, such as the Pittsburgh Sleep Quality Index (PSQI). For example, the sleep quality is determined based on an objective total sleep time and insomnia information, such as described in yet unpublished EP patent application 24169585.7, hereby incorporated by reference. The sleep parameter relates to, for example, sleep time misperception. Sleep time misperception is the difference between the sleep time perceived by the subject and the objective sleep time. The objective sleep time is the measured time the subject is asleep. For the same objective sleep time, subjects that have poor sleep quality tend to perceive that they slept less than subjects that have good sleep quality. For example, the sleep time misperception is determined as described in yet unpublished EP patent application 24169585.7, hereby incorporated by reference.

### MEDICATION INTAKE

According to a seventh aspect of the invention, there is provided the method according to any one of the first aspect, the second aspect, the third aspect, the fourth aspect, the fifth aspect, or the sixth aspect. The medication information comprises medication intake information representative of a medication intake of the subject. The method comprises determining the setting based on the medication intake information.

The influence of the medication on the pathophysiological trait depends on the medication intake. To provide improved SDB therapy to the subject, the setting of the therapy device is determined based on the intake medication. As a result, improved SDB therapy is provided with the therapy device for the subject using medication for treating the SDB.

For example, the medication intake comprises information about when the medication was taken by the subject. For example, the medication intake comprises information about how the dosage of the medication was taken during the day or during several days. For example, the medication intake comprises information about the intake of other medications than the medication that has an influence on the pathophysiological trait. All these examples of medication intake may influence the working of the medication. For example, a timing of the medication intake may determine a period at which the medication is most effective or least effective during a sleep session. For example, the timing of the medication intake determines the time of the half-life of the medication during the sleep session. For example, the medication intake determines the amount of influence on the pathophysiological trait.

Whether and/or when the subject takes the medication may affect the influence of the medication on the pathophysiological trait. For example, if the subject forgets to take the medication or takes the medication at the wrong time, the medication may have less influence on the pathophysiological trait during the sleep session. So by taking the medication intake information into account, the therapy device provides improved SDB therapy to the subject.

In an embodiment, the method comprises generating a message signal adapted to display on a user interface a message relating to a request for the medication intake information. The method comprises receiving a user interface input via the user interface in response to the message. The method comprises determining the setting based on the user interface input.

By providing the message to the user interface, the subject is able to provide the medication intake information via the user interface. As a result, accurate medication intake information is obtained. Optionally, the message is a reminder to the subject to take the medication, which helps to prevent the subject from forgetting to take the medication. For example, the user interface is part of a mobile device, such as a mobile phone or tablet or smart watch. For example, the message signal is sent to the user interface when the user starts to use the respiratory support device. For example, the request asks whether the medication is taken. For example, the request asks when the medication was taken, and/or what dose was taken.

In an embodiment, the method comprises receiving the medication intake information from a medication dispenser; detecting whether the medication intake information from the medication dispenser is inconsistent with the medication intake information from the user interface input; generating an alarm signal in case the medication intake information from the medication dispenser is inconsistent with the medication intake information from the user interface input.

According to this embodiment, the information about the medication intake is obtained in two different ways. The subject is asked for the medication intake information via the user interface. Also, the medication dispenser provides the medication intake information. This way, the medication intake information provided by the subject can be verified by the medication intake information from the medication dispenser. This helps to verify whether the subject is able to work with the user interface. For example, the user interface is too complex for the user, or the user does not have proper attention when using the user interface. If the information is inconsistent, an alarm signal is generated. Based on the alarm signal, the user and/or the doctor may take actions to improve use of the user interface, such as more explanation about the user interface. In case the user is able to use the user interface properly for a certain amount of time, the medication dispenser may no longer be needed. Then, the medication dispenser may be used by another subject.

A medication dispenser is a device that is adapted to provide the medication. For example, the medication dispenser provides a dose of the medication per instance or provides the dose of the medication for a day per instance. For example, the medication dispenser releases a pill per instance, or opens part of the medication dispenser allowing the subject to take the pill from the medication dispenser per instance. For example, the medication dispenser comprises a container for containing pills. For example, the medication dispenser comprises an inhaler or a nebulizer to provide the medication. The medication dispenser is adapted to provide the medication intake information.

In an embodiment, the method comprises determining whether at least one prescribed medication intake did not occur based on the medication intake information. The method comprises determining, in response to determining at least one prescribed medication intake did not occur, whether the influence of the medication on the pathophysiological trait is below a threshold level. The method comprises determining a change in the setting in response to the influence being below the threshold level. The method comprises determining no change in the setting in response to the influence not being below the threshold level.

The subject may not take the medication as prescribed. For example, the subject forgets to take the medication, or the subject did not timely obtain a resupply of the medication. Not taking the medication as prescribed reduces the influence of the medication on the pathophysiological trait. However, in case the medication is not taken only once or only few times, the reduction of the influence may be small. If the reduction of the influence is small, there is no need to adjust the setting, as this would not affect the therapy significantly. In case the reduction of the influence becomes below a threshold, the reduction of the influence is substantial. To compensate for the substantial reduction of the influence, the setting is adjusted. For example, some medications require a long intake period to build up sufficiently to provide the therapeutic effect. Such medication may maintain the built up concentration even if the subject forgets to take the medication once or a few times. So for those medications, the influence comes below the threshold level if the subject does not take the medication for a large number of occasions. These medications may have a large half-life. For other medications, such as with a half-life of several hours, the influence reduces substantially when the subject does not take the medication as prescribed once or twice.

In an embodiment, the medication intake information comprises information about a medication intake time. The method comprises determining the setting of the therapy device based on the medication intake time.

The time the subject takes the medication may be important for the influence of the medication on the pathophysiological trait during the sleep session, especially for medications with a short half-life. For example, the half-life is less than the duration of the sleep session. If the subject takes the medication at the prescribed time, the medication is able to provide an optimal therapeutic effect during the sleep session. If the subject takes the medication too early, the medication may stop providing the therapeutic effect before the end of the sleep session. The setting is, for example, adjusted for the last part of the sleep session to compensate for the reduced therapeutic effect. If the subject takes the medication too late, the medication may not provide the therapeutic effect at the start of the sleep session. The setting is, for example, adjusted for the first part of the sleep session to compensate for the lack of the therapeutic effect.

In an embodiment, the medication information comprises a medication intake history representative of a history of an intake of the medication by the subject during a period in the past. The method comprises estimating an effectiveness of the medication based on the medication intake history. The method comprises determining the setting of the therapy device based on the effectiveness of the medication.

According to this embodiment, the history of the intake of the medication is used to estimate the effectiveness of the medication. The setting of the therapy device is based on the estimated effectiveness. For example, if the history indicates the subject took the medication as prescribed over a period of time, the effectiveness of the medication is estimated to be high. As a result, the setting of the therapy is set accordingly, for example to provide SDB therapy at a low intensity level. For example, if the history indicates the subject forgot to take the medication as prescribed or took the medication irregularly over a period of time, the effectiveness of the medication is estimated to be low. As a result, the setting of the therapy is set accordingly, for example to provide SDB therapy at a high intensity level.

In an embodiment, the method comprises receiving the medication intake information from a medication dispenser.

When the subject interacts with the medication dispenser to take a dose of the medication, the medication dispenser is able to generate the medication intake information. For example, the medication dispenser detects when the subject retrieves or does not retrieve the medication from the medication dispenser.

In an embodiment, the medication information is indicative of a first medication and a second medication used by the subject. The method comprises determining a first setting of the therapy device based on the trait information representative of the influence of the first medication on the pathophysiological trait. The method comprises determining a second setting of the therapy device based on the trait information representative of the influence of the second medication on the pathophysiological trait. The method comprises determining the setting of the therapy device based on the first setting and the second setting.

In this embodiment, the subject uses the first medication and the second medication to influence the pathophysiological trait. The first medication and the second medication may influence the pathophysiological differently, e.g., differently over time, differently over the sleep session, affecting the severity of the SDB differently. The setting is based on the influence of both the first medication and the second medication. For example, the setting is an average of the first setting and the second setting. For example, the setting is the most intensive of the first setting and the second setting. For example, the setting relates to multiple therapy parameters, wherein the first setting relates to a different therapy parameter than the second setting.

In an embodiment, the method comprises determining a desired effect profile for at least one of the first medication and the second medication based on the first setting and the second setting. The desired effect profile is representative of a desired effect during a sleep session of at least one of the first medication and the second medication. The method comprises generating a profile output signal based on the desired effect profile.

Due to changes in the effectiveness of the first medication and/or the second medication during the sleep session, the SDB therapy may not be provided properly during some parts of the sleep session. For example, the SDB therapy is provided at a very high intensity disturbing the sleep of the subject. For example, the SDB therapy is not able to reduce the SDB events sufficiently. The desired effect profile is determined based on the first setting and the second setting. The desired effect profile represents a desired effect of the first medication and/or second medication to cause the combined effect with the first setting and the second setting to provide improved SDB therapy. A signal representative of the desired effect profile is output via the profile output signal. Based on the profile output signal, for example, a pharmaceutical manufacturer is able to adjust the first medication and/or the second medication to obtain the desired effect profile. For example, the pharmaceutical manufacturer is able to adapt a coating of a pill with the medication to adjust the timing of the release the medication. For example, the doctor is able to prescribe a different medication or an additional medication to achieve the desired effect profile.

### COMPUTER PROGRAM FOR MEDICATION INTAKE

According to an eight aspect of the invention, there is provided a computer program product comprising instructions which, when executed by a processor, causes to carry out a computer-implemented method. The computer-implemented method comprises generating a message signal adapted to display on a user interface a message relating to a request for medication intake information. The medication intake information is representative of a medication intake of the subject. The computer-implemented method comprises receiving a user interface input via the user interface in response to the message; and providing the user interface input as medication information for use in the method according to any one of the first aspect, the second aspect, the third aspect, the fourth aspect, the fifth aspect, the sixth aspect, or the seventh aspect.

### DEVICES

According to a nineth aspect of the invention, there is provided a computer program product, comprising instructions which, when executed by a processor, cause the method according to any one of the first aspect, the second aspect, the third aspect, the fourth aspect, the fifth aspect, the sixth aspect, or the seventh aspect to be carried out.

According to a tenth aspect of the invention, there is provided a computer-readable storage medium comprising instructions which, when executed by a processor, causes the method according to any one of the first aspect, the second aspect, the third aspect, the fourth aspect, the fifth aspect, the sixth aspect, or the seventh aspect to be carried out.

According to an eleventh aspect of the invention, there is provided a respiratory support device adapted to provide a pressurized airflow to a subject. The respiratory support device comprises an air pressure source adapted to generate the pressurized airflow; an outlet connectable to a patient interface to provide the pressurized airflow to the subject; and a processor configured to execute a computer program product comprising instructions which, when executed by the processor, causes the method according to any one of the first aspect, the second aspect, the third aspect, the fourth aspect, the fifth aspect, the sixth aspect, or the seventh aspect to be carried out. The respiratory support device is the therapy device.

In an embodiment, the setting comprises a setting of the air pressure source. The processor is configured to control the air pressure source to set the pressurized airflow based on the setting.

According to a twelfth aspect of the invention, there is provided a mandibular advancement device (MAD) comprising: a first member adapted to engage a maxilla of the subject; a second member adapted to engage a mandible of the subject; and an adjustment device adapted to adjust an offset between the first member and the second member to advance the mandible relative to the maxilla. The adjustment device comprises an actuator. The actuator is adapted to receive the instruction signal generated according to the method of the first aspect. The MAD is the therapy device. The actuator is adapted to adjust the offset between the first member and the second member based on the instruction signal.

According to a thirteenth aspect of the invention, there is provided a positional therapy device, comprising: a body position sensor adapted to generate a positional signal representative of a body position of the subject; a stimulator adapted to provide a stimulus to the subject; and a controller configured to control the stimulator to provide the stimulus to the subject in response to the positional signal being representative of the body position being in a supine position. The controller comprises a processor configured to execute a computer program product comprising instructions which, when executed by the processor, causes the method according to any one of the first aspect, the second aspect, the third aspect, the fourth aspect, the fifth aspect, the sixth aspect, or the seventh aspect to be carried out. The positional therapy device is the therapy device.

### BRIEF DESCRIPTION OF THE DRAWINGS

Exemplary embodiments will now be described, by way of example only, with reference to the following figures, in which:
FIG. 1 depicts a first aspect of the invention;
FIG. 2 depicts medication information for use in the first aspect of the invention;
FIG. 3 depicts information about a PK/PD model for use in the first aspect of the invention;
FIG. 4 depicts medication information for use in the first aspect of the invention;
FIG. 5 depicts a respiratory support device according to the first aspect of the invention;
FIG. 6 depicts an MAD according to the first aspect of the invention;
FIG. 7 depicts a positional therapy device according to the first aspect of the invention;
FIG. 8 depicts a detail of the positional therapy device of FIG. 7.
FIG. 9 depicts four pathophysiological traits that have an influence on SDB.
FIG. 10 depicts the processor according to a second aspect of the invention;
FIG. 11 depicts a relationship between fluid shift and AHI for use in the second aspect of the invention;
FIG. 12 depicts a second embodiment of the second aspect of the invention;
FIG. 13 depicts a relationship between neck fat mass and AHI for use in the second aspect of the invention;
FIG. 14 depicts the processor according to a third aspect of the invention;
FIG. 15 depicts a first embodiment according to the third aspect of the invention;
FIG. 16 depicts a third embodiment according to the third aspect of the invention;
FIG. 17 depicts an eight embodiment according to the third aspect of the invention;
FIG. 18 depicts information for determining a loop gain metric according to the nineth embodiment of the third aspect of the invention;
FIG. 19 depicts the processor according to a fourth aspect of the invention;
FIG. 20 depicts a first embodiment according to the fourth aspect of the invention;
FIG. 21 depicts a heart rate according to a fourth embodiment of the fourth aspect of the invention;
FIG. 22 depicts the processor according to a fifth aspect of the invention;
FIG. 23 depicts a first embodiment according to a sixth aspect of the invention;
FIG. 24 depicts a second embodiment according to the sixth aspect of the invention;
FIG. 25 depicts a first embodiment according to the eighth aspect of the invention;
FIG. 26 depicts a second embodiment according to the eighth aspect;
FIG. 27 depicts a third embodiment according to the eighth aspect;
FIG. 28 depicts obtaining information from the subject for use in any one of the aspects of the invention.

### DETAILED DESCRIPTION OF EMBODIMENTS

It should be understood that the detailed description and specific examples, while indicating exemplary embodiments of the devices and methods, are intended for purposes of illustration only and are not intended to limit the scope of the invention. These and other features, aspects, and advantages of the devices and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

### GENERAL ASPECTS OF THE INVENTION

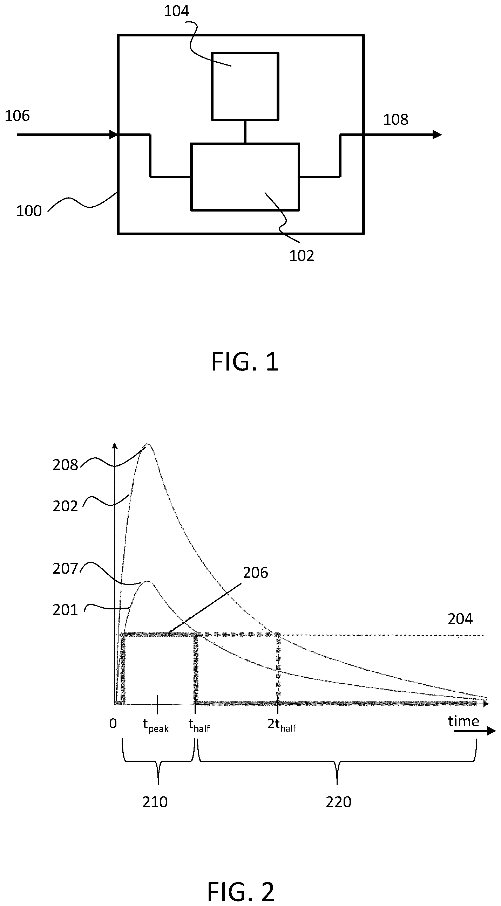

FIG. 1 depicts processor 100 according to a first aspect of the invention. The processor 100 comprises a processing unit 102 and a memory 104 connected to the processing unit 102. The memory 104 is a computer-readable storage medium that stores a computer program product. The computer program product comprises instructions which, when executed by the processor 100, causes a method according to the first embodiment to be carried out.

The method is a computer-implemented method for determining a setting 108 of a therapy device adapted to provide sleep disordered breathing (SDB) therapy to a subject. The method comprises receiving medication information 106 representative of medication used by the subject. The method comprises determining trait information based on the medication information 106. The trait information is representative of an influence of the medication on a pathophysiological trait of the subject. The pathophysiological trait has an influence on the SDB of the subject. The method comprises determining the setting 108 of the therapy device based on the trait information.

The medication information 106 comprises information about a half-life of the medication, or a dosage of the medication, or a tolerance to the medication, or an effectiveness of the medication, or a duration of use of the medication, or a pharmacokinetic (PK) model, or a pharmacodynamic (PD) model, or a pharmacokinetic/ pharmacodynamic (PK/PD) model.

FIG. 2 depicts medication information 106 for use in the first aspect of the invention. The medication information 106 is about the half-life and the dosage. Line 201 depicts the concentration of the medication in the subject versus the time. At t=0, the subject takes the medication and goes to bed. After some time, the concentration rises to above a therapeutic level 204. This causes the medication to cause a therapeutic effect 206 on the subject. At tₚₑₐₖ, the concentration reaches the peak concentration 207. The peak concentration 207 causes the largest therapeutic effect 206 on the subject. After tₚₑₐₖ, the concentration decreases. At the half-life, t_{half}, the concentration has decreased to the therapeutic level 204. After t_{half}, the concentration is not high enough to provide any therapeutic effect 206. Line 202 depicts the concentration relating to a double dose of the medication taken by the subject when going to bed. As shown in FIG 2, the concentration rises more quickly and reaches a higher peak concentration 208. After the peak concentration 208, the concentration decreases but stays above the therapeutic level 204 till twice the half time, 2t_{half}. Depending on the medication, the concentration may be below the therapeutic level 204 before the half-life, or may remain above the therapeutic level 204 after the half-life or after twice the half-life. Depending on the medication, the concentration may remain at the peak concentration for a substantially long time, such as multiple hours.

The medication has an influence on the pathophysiological trait of the subject when the concentration is above the therapeutic level 204. When the concentration is below the therapeutic level 204, the medication has a low or no influence on the pathophysiological trait.

Based on the medication information 106 as shown in FIG. 2, the trait information can be determined during a first time period 210 and during a second time period 220. The first time period 210 is when the concentration is above the therapeutic level 204. The second time period 220 is when the concentration is below the therapeutic level 204. During the first time period 210 the influence of the medication on the pathophysiological trait is larger than during the second time period 220. The setting 108 of the therapy device is determined to provide the SDB therapy having a therapy parameter. The therapy parameter has a first value during the first time period 210 and has a second value during the second time period 220. The first value is different from the second value. The first time period 210 and the second time period 220 are in the same sleep session of the subject.

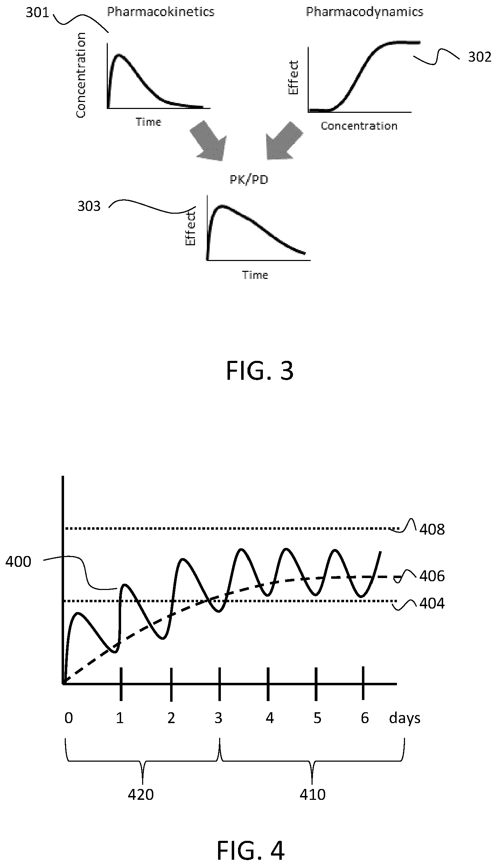

FIG. 3 depicts information about a PK/PD model for use in the first aspect of the invention. Graph 301 depicts the outcome of a pharmacokinetic (PK) model for the medication. The pharmacokinetic model outputs the concentration of the medication as a function of time. As shown in graph 301, the concentration increases till a peak is reached. After the peak, the concentration gradually decreases. Graph 302 depicts the outcome of a pharmacodynamic (PD) model. The pharmacodynamic model outputs the effect of the medication on the pathophysiological trait as a function of the concentration. Graph 302 indicates that the effect increases with increasing concentration. Graph 303 combines the information from the pharmacokinetic model and the pharmacodynamic model to provide an effect of the medication on the pathophysiological trait as a function of time. As shown in graph 303, the effect reaches a peak effectiveness after which the effectiveness gradually decreases. Based on the medical information of one or more of graph 301, graph 302, and graph 303, the trait information is determined. The trait information indicates that the pathophysiological trait is most improved at the time of the peak effectiveness. Based on the trait information, the setting 108 of the therapy device is determined. For example, the setting 108 of the therapy is to provide SDB therapy with a lower intensity around the time of the peak effectiveness than during other times in the sleep session.

FIG. 4 depicts medication information 106 for use in the first aspect of the invention. The graph in FIG. 4 depicts the concentration 400 of the medication as a function of time. The time is on a scale of multiple days, from day 0 to day 6. Every day, the subject administers a dose of the medication. This increases the concentration 400 till it reaches a peak value that day. After the peak value, the concentration 400 decreases till the subject administers another dose the next day. The concentration 400 gradually increases because of the multiple doses over the multiple days. As a result of the increase in concentration 400, the therapeutic effect 406 of the medication increases over multiple days. The concentration reaches the therapeutic level 404 and causes the therapeutic effect 406 on the pathophysiological trait. The concentration 400 should remain below the toxic level 408 to prevent toxification or excessive side effects.

Based on the medication information 106 as shown in FIG. 4, the trait information can be determined during a first time period 410 and during a second time period 420. The first time period 410 is when the effect is above the therapeutic level 404, from day 3 onwards. The second time period 420 is when the effect is below the therapeutic level 404, between day 0 and day 3. During the first time period 410 the influence of the medication on the pathophysiological trait is larger than during the second time period 420. The setting 108 of the therapy device is determined to provide the SDB therapy having a therapy parameter. The therapy parameter has a first value during the first time period 410 and has a second value during the second time period 420. The first value is different from the second value. The first time period 410 and the second time period 420 are in different sleep sessions of the subject, i.e., the sleep sessions on days 0 to day 3, and the sleep sessions on days 3 onwards.

For example, the therapy parameter relates to at least one of an intensity level of the SDB therapy, a rate of change of the intensity level of the SDB therapy, a range of the intensity level of the SDB therapy, or a difference in intensity level of the SDB therapy between inhalation and exhalation of the subject. The therapy parameters are further disclosed in the further embodiments. Optionally, the processor 100 is adapted to adjust the therapy device according to the setting 108 to provide the SDB therapy having the first value during the first time period 410 and having the second value during the second time period 420.

### THERAPY DEVICES

Various therapy devices are available to treat SDB. The setting 108 as determined by the processor 100 may be used with any of those therapy devices. Any one of the three therapy devices described below may be used in the first aspect of the invention.

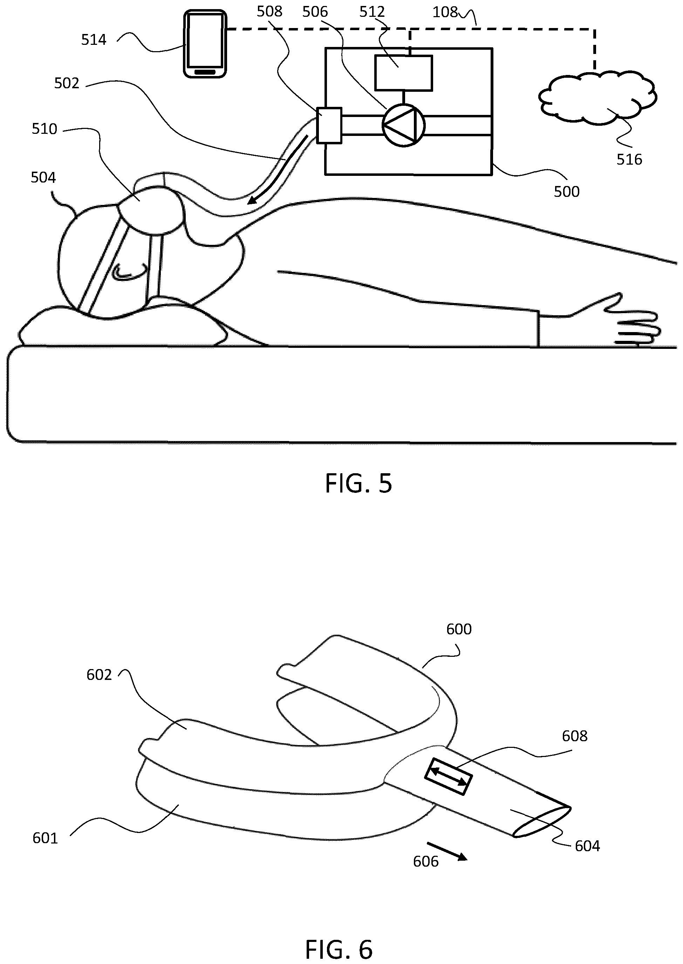

As depicted in FIG. 5, the first therapy device is a respiratory support device 500 adapted to provide a pressurized airflow 502 to a subject 504. The respiratory support device 500 comprises an air pressure source 506, and an outlet 508. The air pressure source 506 is adapted to generate the pressurized airflow 502. The outlet 508 is connectable to a patient interface 510 to provide the pressurized airflow 502 to the subject 504. The processor 100 is configured to execute a computer program product comprising instructions which, when executed, causes the method according to the first embodiment to be carried out.

For example, the processor 100 is arranged outside the respiratory support device 500, such as in a mobile device 514, or in the cloud 516. The respiratory support device 500 has a controller 512 configured to receive the determined setting 108 via wired or wireless transmission, such as LAN, Wi-Fi, or Bluetooth. The controller 512 controls the respiratory support device 500 according to the setting 108. In another example, the controller 512 of the respiratory support device 500 comprises the processor 100.

In an embodiment, the setting 108 comprises a setting of the air pressure source 506. The processor 100 is configured to control the air pressure source 506 to set the pressurized airflow 502 based on the setting 108. For example, the controller 512 controls the air pressure source 506 to adjust the pressure or pressure profile of the pressurized airflow 502. For example, the therapy parameter relates to a pressure or a pressure profile of the pressurized airflow 502.

As depicted in FIG. 6, the second therapy device is a mandibular advancement device (MAD) 600. The MAD 600 comprises a first member 601, a second member 602 and an adjustment device 604. The first member 601 adapted to engage a maxilla of the subject. The second member 602 adapted to engage a mandible of the subject 504. The adjustment device 604 is adapted to adjust an offset 606 between the first member 601 and the second member 602 to advance the mandible relative to the maxilla.

In an embodiment, the processor 100 generates an instruction signal representative of an instruction for adjusting the offset 606 with the adjustment device 604. The instruction signal is based on determining the setting 108 of the therapy device based on the trait information and the medication information 106. Based on the instruction signal, the subject 504 or a medical professional is able to adjust the offset 606 to the proper value. For example, the instruction signal instructs to set the offset 606 to certain number of millimeters.

In an embodiment, the adjustment device 604 comprises an actuator 608. The actuator 608 is adapted to receive the instruction signal. The processor 100 provides the instruction signal to the actuator 608. The actuator 608 is adapted to adjust the offset 606 between the first member 601 and the second member 602 based on the instruction signal. For example, the setting 108 comprises a setting of the offset 606, or the speed of changing the offset 606. For example, the therapy parameter relates to the offset 606. For example, the MAD 600 comprises the processor 100. For example, the processor 100 is in a mobile device or in the cloud. The MAD 600 is adapted to receive the determined setting 108 via wireless connection, such as Wi-Fi or Bluetooth. For example, the MAD 600 is adapted to receive the determined setting 108 via docking the MAD 600 in a docking station. The docking station is able to transfer the setting 108 to the MAD 600. For example, the docking station is adapted to charge a battery of the MAD 600. The battery is adapted to provide electric power to the actuator 608.

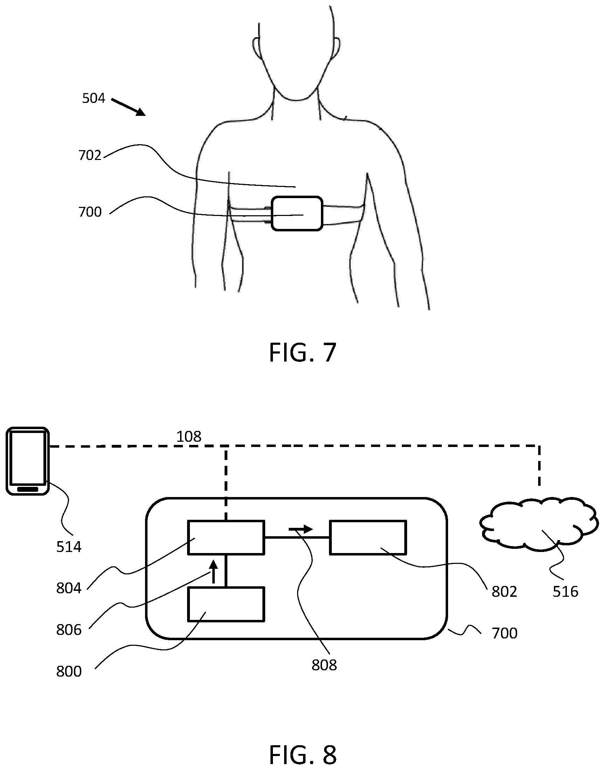

As depicted in FIG. 7, the third therapy device is a positional therapy device 700. A detail of the positional therapy device 700 is depicted in FIG. 8. The positional therapy device 700 is attached to the chest of the subject 504 using a belt. Alternatively, the positional therapy device 700 is adapted to be attached to the neck, or back, or head of the subject 504.

The positional therapy device 700 comprises a body position sensor 800, a stimulator 802, and a controller 804. The body position sensor 800 is adapted to generate a positional signal 806 representative of a body position of the subject 504. The controller 804 is adapted to generate a stimulation signal 808 in response to the positional signal 806. The stimulator 802 adapted to provide a stimulus to the subject 504 based on the stimulation signal 808. The controller 804 is configured to control the stimulator 802 to provide the stimulus to the subject 504 in response to the positional signal 806 being representative of the body position being in a supine position.

The processor 100 is configured to execute a computer program product comprising instructions which, when executed, causes the method according to the first embodiment to be carried out. For example, the processor 100 is arranged outside the positional therapy device 700, such as in a mobile device 514, or in the cloud 516. The positional therapy device 700 has the controller 804 configured to receive the determined setting 108 via wired or wireless transmission, such as LAN, Wi-Fi, or Bluetooth. The controller 804 controls the positional therapy device 700 according to the setting 108. In another example, the controller 804 of the positional therapy device 700 comprises the processor 100.

In an embodiment, the setting 108 comprises a setting of the stimulator 802. The controller 804 is configured to set an intensity level of the stimulus provided by the stimulator 802 based on the setting 108. For example, the stimulator 802 is a vibrator. The intensity level of the stimulus is set by setting 108 an amplitude of a vibration and/or a frequency of a vibration and/or a number of vibrations and/or a duration of vibrations generated by the vibrator.

### INTRODUCTION FOUR PATHOLOGICAL TRAITS

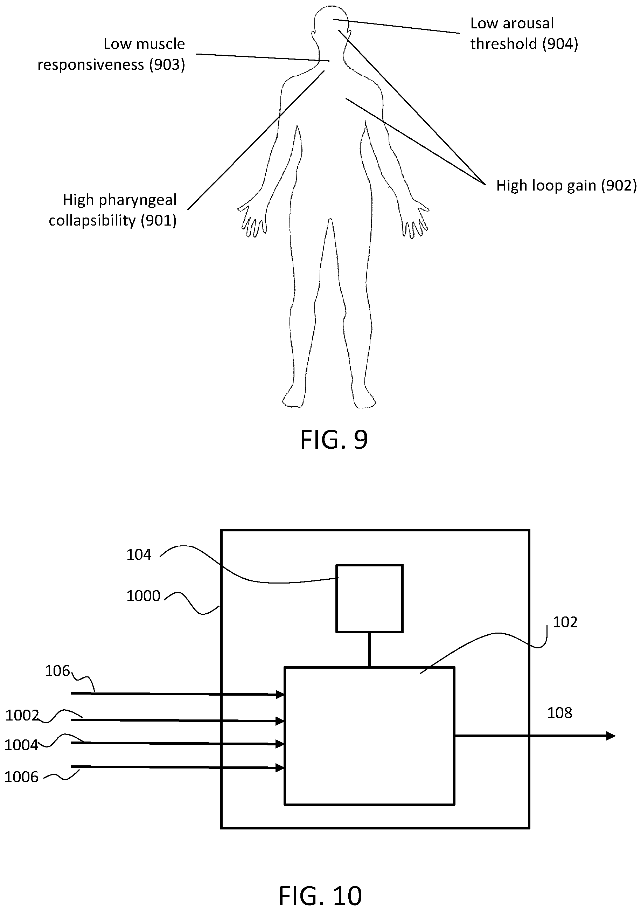

The study of Perger et.al, as mentioned in the background, indicates four pathophysiological traits that have an influence on SDB. FIG. 9 depicts the four pathological traits. These four pathophysiological traits are a high pharyngeal collapsibility 901, a high loop gain 902, a low muscle responsiveness 903, and a low arousal threshold 904. The study indicates that medication is available for each of the four pathophysiological traits. The following aspects of the invention make use of the insights by the inventors that by using the trait information representative of an influence of the medication on one of these four pathophysiological traits, an improved setting 108 of the therapy device is obtained to provide improved SDB therapy to the subject 504 who uses medication that targets that pathophysiological trait.

The various embodiments of each of the following aspects, i.e. the second - fifth aspect, of the inventions make use of the trait information representative of the influence of the medication on these four pathophysiological traits.

### HIGH PHARYNGEAL COLLAPSIBILITY

FIG. 10 depicts the processor 1000 according to a second aspect of the invention. The processor 1000 comprises a processing unit 102 and a memory 104 connected to the processing unit 102. The memory 104 is a computer-readable storage medium that stores a computer program product. The computer program product comprises instructions which, when executed by the processor 1000, causes a method according to the second embodiment to be carried out. The second aspect is the same as the first aspect, except for the following. For example, the processor 1000 is the same as processor 100, except for the following.

In an embodiment, the invention according to the second aspect is combined with any of the embodiments according to the first aspect of the invention.

In the second aspect, the pathophysiological trait comprises a high pharyngeal collapsibility 901.

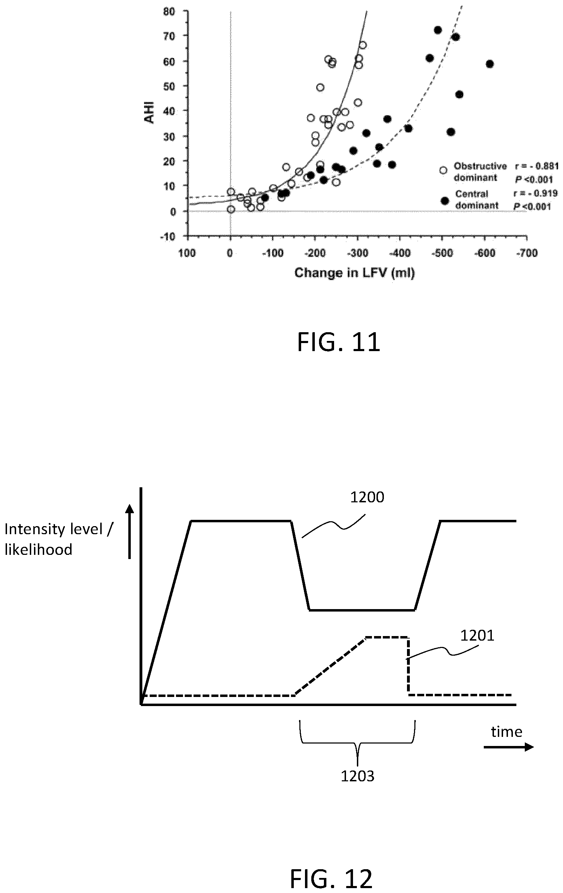

The study by Dai Yumino, et.al "Nocturnal Rostral Fluid Shift: A Unifying Concept for the Pathogenesis of Obstructive and Central Sleep Apnea in Men With Heart Failure", Circulation, 2010 April 13, 121(14): 1598-605, indicates depicts a relationship between the fluid shift and AHI as shown in FIG. 11. The x-axis indicates the change in leg volume fluid (LVF), and the y-axis indicates the AHI. The change in LVF is negative to indicate the amount of fluid leaving the legs and moving towards the upper body. The higher the fluid shift of fluid from the less towards the upper body, the worse the AHI becomes.

By estimating the amount of fluid shift and/or the rate of the fluid shift, the severity of the high pharyngeal collapsibility can be estimated.

In a first embodiment, the medication comprises a diuretic. The method comprises estimating a fluid shift in the subject 504 during a sleep session caused by the diuretic based on the medication information 106. The method comprises determining the trait information based on the fluid shift.

By taking a diuretic, water is shifted from the body. As a result, water is removed from the upper airway, and less water remains in the subject 504 to shift towards the upper airway. The estimation of the fluid shift takes into account, for example, the amount of water shifted by the diuretic or the time the diuretic needs to shift the water. For example, the estimation of the fluid shift takes into account when the diuretic has worn out. Then a remaining fluid shift from the lower body may again cause an accumulation of fluid at the upper airway.

For example, the setting 108 is determined to be at a high intensity level in case a certain amount of fluid, for example 150 ml, has shifted from the lower body to the upper body, and to be at a low intensity before the certain amount of fluid has shifted. For example, the setting 108 is determined to be at a low intensity level in case the diuretic caused a certain amount of fluid, for example 150 ml, to shift and to be at a high intensity before the diuretic causes the certain amount to shift. For example, in case the therapy device is a respiratory support device 500, the pressure of the pressurized air is changed linearly with the fluid shifted.

FIG. 12 depicts a second embodiment of the second aspect. Line 1200 shows the intensity level of the SDB therapy over time. Line 1201 shows a likelihood the subject 504 needs to urinate. In the second embodiment, the method comprises estimating a urinary time period 1203 in the sleep session based on the medication information 106. During the urinary time period 1203 the subject 504 has likely a need to urinate. The likelihood as indicated by line 1201 has increased from a low value to a high value. The method comprises determining the setting 108 of the therapy device to provide the SDB therapy at a lower intensity level during the urinary time period than before the urinary time period, as shown with line 1200. For example, the method uses the amount of fluid shift caused by the diuretic to estimate the likely need to urinate. The urinary time period 1203 ends when the subject goes to the toilet. After the subject 504 returns to bed after urinating, the likelihood is reduced to the low value. The intensity level of the SDB therapy is increased to the level previous to the urinary time period 1203.

In a third embodiment, the method comprises receiving blood pressure information 1002 representative of a blood pressure of the subject 504. The method comprises determining the trait information based on the blood pressure information 1002.

In a fourth embodiment, the medication comprises a weight loss medication. The method comprises providing a weight loss estimation based on the medication information 106. The method comprises determining the trait information based on the weight loss estimation.

In a fifth embodiment, the method comprises receiving neck circumference information or body weight information 1004 representative of a body weight of the subject 504. The method comprises determining the trait information based on the neck circumference information or the body weight information 1004.

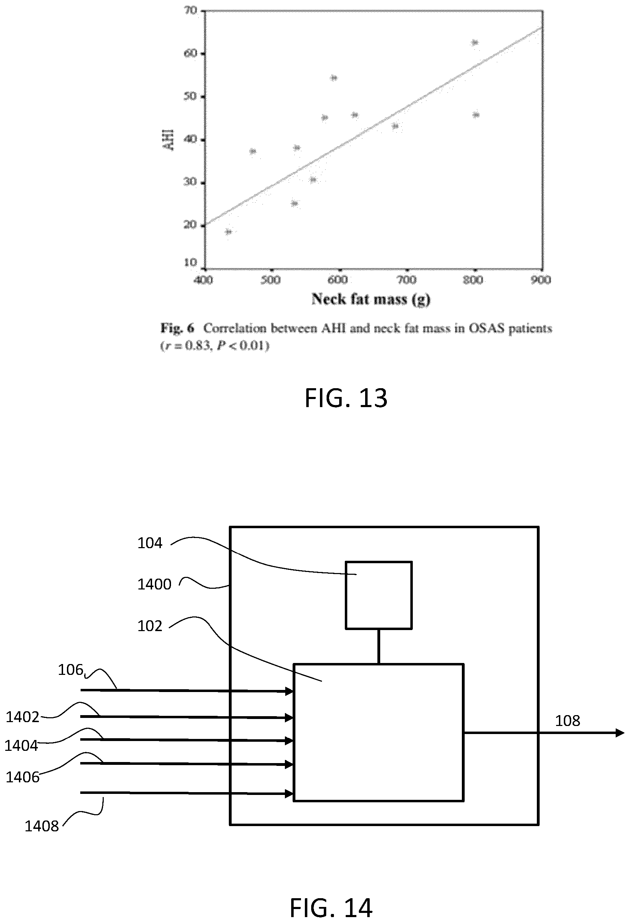

The study by Bruno, et.al, "Dual-Energy X-Ray Absorptiometry Analysis of Body Composition in Patients Affected by OSAS." European Archives of Oto-Rhino-Laryngology 266, no. 8 (August 2009): 1285-90. https://doi.org/10.1007/s00405-008-0844-0, shows a correlation between neck fat mass and AHI as depicted in FIG. 13. A similar correlation exists between body weight and AHI. By taking the taking the neck circumference or the body weight into account, a more accurate estimation of the high pharyngeal collapsibility can be made.

In a sixth embodiment, the method comprises receiving AHI information 1006 representative of an Apnea Hypopnea Index (AHI) of the subject 504 during a sleep session without SDB therapy. The method comprises determining the trait information based on the AHI information 1006. The AHI information 1006 helps to estimate the weight loss caused by the weight loss medication. As the weight loss contributes to the high pharyngeal collapsibility, the AHI information 1006 improves the trait information.

In an embodiment, the medication comprises a nasal decongestant.

### HIGH LOOP GAIN

FIG. 14 depicts the processor 1400 according to a third aspect of the invention. The processor 1400 comprises a processing unit 102 and a memory 104 connected to the processing unit 102. The memory 104 is a computer-readable storage medium that stores a computer program product. The computer program product comprises instructions which, when executed by the processor 1400, cause a method according to the third embodiment to be carried out. The third embodiment is the same as the first embodiment or as the second embodiment, except for the following. For example, the processor 1400 is the same as the processor 100, except for the following.

In an embodiment, the invention according to the third aspect is combined with any of the embodiments according to the first aspect, or the second aspect of the invention.

According to the third aspect, the pathophysiological trait comprises a high loop gain.

Medications such as acetazolamide, sulthiame, and topiramate are all carbonic anhydrase enzyme inhibitors (CAI). By increasing HCO3 excretion and causing metabolic acidosis, CAIs stimulate ventilation, by lowering the loop gain. CAIs lower the loop gain mainly by reducing the ventilatory response to arousals and increasing the feedback system's cycling period. Some CAIs may further act by reducing the apneic threshold, thus increasing the CO2 reserve. Both mechanisms contribute to the regularization of breathing and preventing apnea exacerbation as a response to sudden changes in ventilation. For example, the medication comprises a carbonic anhydrase enzyme inhibitor (CAI).

For example, the setting 108 is set to provide with the respiratory support device 500 the pressurized airflow 502 at a higher pressure in case the medication has less influence, for example due to the medication wearing off during the sleep session, or due to tolerance to the medication. For example, the setting 108 is set to provide with the respiratory support device 500 the pressurized airflow 502 at pressure high enough to prevent residual SDB events. Without any residual SDB events, ventilation overshoots and resulting unstable breathing are prevented.

The processor 1400 is configured to receive respiratory information 1402, eupneic information 1404, oxygen saturation information 1406, and/or sleep stage information 1408.

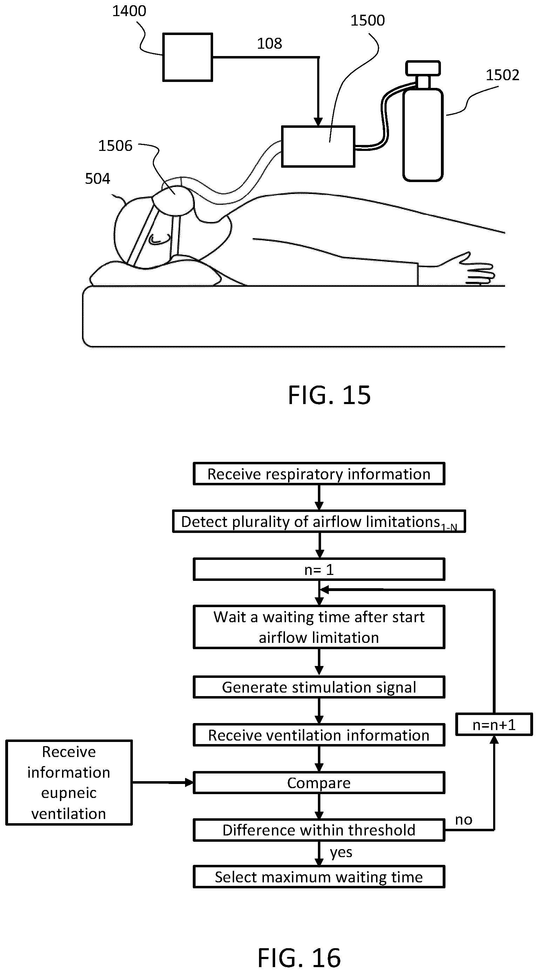

FIG. 15 depicts a first embodiment according to the third aspect. The therapy device 1500 is adapted to provide oxygen to the subject 504. The method comprises determining the setting 108 of the therapy device 1500 comprises determining a flow rate of the oxygen to the subject 504 based on the trait information.

The therapy device 1500 comprises a connector to connect to a cannister of oxygen 1502. The therapy device 1500 is adapted to receive the oxygen from the cannister 1502 and to provide the oxygen to the subject 504 via a patient interface 1506. The therapy device 1500 is adapted to provide the oxygen with the flow rate as determined by the setting 108. Optionally, the therapy device 1500 is adapted to mix the oxygen with ambient air before providing the oxygen to the subject 504.

In an embodiment, the therapy device 1500 is adapted to provide carbon dioxide to the subject 504. The method comprises determining the setting 108 of the therapy device comprises determining a flow rate of the carbon dioxide to the subject 504 based on the trait information.

Similar to FIG. 15, the therapy device comprises a connector to connect to a cannister. The cannister contains carbon dioxide. The therapy device is adapted to receive the carbon dioxide from the cannister and to provide the carbon dioxide to the subject 504 via a patient interface. The therapy device is adapted to provide the carbon dioxide with the flow rate as determined by the setting 108. Optionally, the therapy device is adapted to mix the carbon dioxide with ambient air before providing the carbon dioxide to the subject 504.

In a second embodiment, the method comprises receiving respiratory information 1402 representative of respiration of the subject 504 during a sleep session, as depicted in FIG. 14. The method comprises determining a beginning of an airflow limitation based on the respiratory information 1402. The method comprises determining whether the airflow limitation is still occurring after a maximum waiting time. In case the airflow limitation is still occurring after the maximum waiting time, the method comprises generating a stimulation signal. The stimulation signal is adapted to cause a stimulator to provide a stimulus to the subject 504. The stimulus is adapted to cause arousal of the subject 504. The stimulus causes an arousal of the subject, and activates the non-chemical drive. As a result, the ventilation does not become instable and does not create a series of SDB events.

FIG. 16 depicts a third embodiment according to the third aspect. The method comprises receiving information about a eupneic ventilation of the subject 504. The processor 1400 is configured to receive the eupneic ventilation information 1404 as depicted in FIG. 14. The method comprises receiving respiratory information 1402 representative of respiration of the subject 504. The method comprises determining the maximum waiting time. This is done as follows. First a plurality of airflow limitations is detected based on the respiratory information 1402. A waiting time is waited after a start of each airflow limitation. The waiting time is different for each airflow limitation. A stimulation signal is generated when the waiting time expires. The stimulation signal is adapted to cause a stimulator to provide a stimulus to the subject 504. Ventilation information is received. The ventilation information is representative of a ventilation of the subject 504 after the stimulus. The ventilation information is compared with the eupneic ventilation. As the maximum waiting time, the waiting time is selected corresponding to the ventilation of the subject 504 being different from the eupneic ventilation within a threshold.

In a fourth embodiment, the therapy device comprises a respiratory support device 500 adapted to provide pressurized airflow 502 to the subject 504. The therapy device comprises the stimulator. The stimulation signal is adapted to cause the respiratory support device 500 to provide the pressurized airflow 502 to the subject 504 in a series of pulses.

In a fifth embodiment, the therapy device comprises the respiratory support device 500 adapted to provide pressurized airflow 502 to the subject 504. The method comprises determining the trait information during a first time period 210 and during a second time period 220. During the first time period 210 the influence of the medication on the high loop gain is larger than during the second time period 220. The method comprises determining the setting 108 the therapy device to provide the pressurized airflow 502 with an inspiration pressure and an expiration pressure. A difference between the inspiration pressure and the expiration pressure is larger during the second time period 220 than during the first time period 210.

In a sixth embodiment, the therapy device comprises the respiratory support device 500 adapted to provide pressurized airflow 502 to the subject 504. The method comprises receiving respiratory information 1402 representative of respiration of the subject 504 during a sleep session. The method comprises determining an SDB event based on the respiratory information 1402. The method comprises determining the setting 108 of the therapy device to provide the pressurized airflow 502 with an inspiration pressure and an expiration pressure. A difference between the inspiration pressure and the expiration pressure is larger after the SDB event than before the SDB event.

In a seventh embodiment, the processor 1400 is configured to receive oxygen saturation information representative of an oxygen saturation of the subject 504, as depicted in FIG. 14. The processor 1400 is configured to determine the trait information based on the oxygen saturation information 1406.

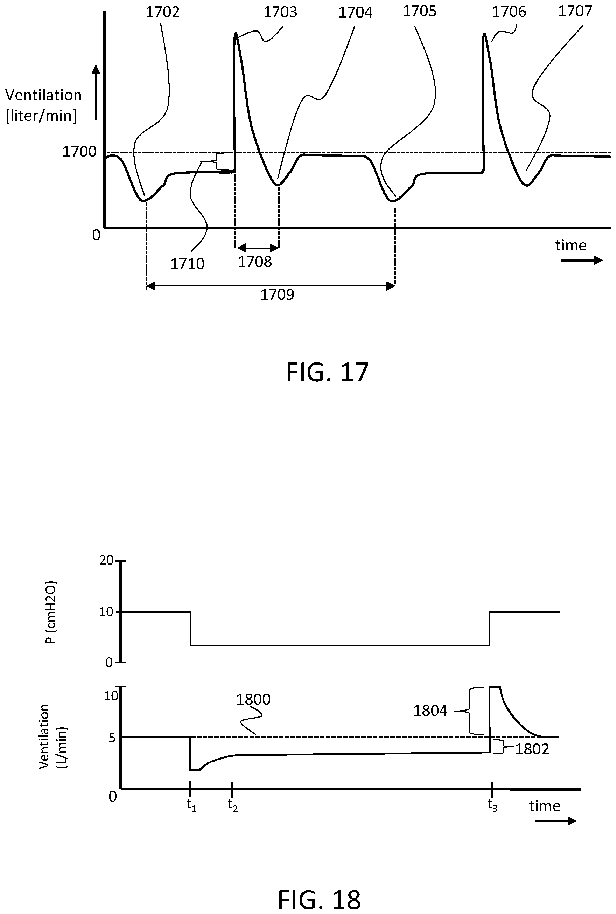

FIG. 17 depicts an eighth embodiment according to the third aspect. FIG. 17 depicts the ventilation as a function of time. At t=0, the ventilation is eupneic ventilation, as indicated with line 1700. Then an airflow limitation occurs, in this case an obstruction 1702 of the ventilation. The subject recovers partly from the obstruction 1702, but the ventilation remains below the eupneic ventilation, as indicated with disturbance 1710. The disturbance 1710 causes the chemical drive to increase till the combination of the chemical drive and the non-chemical drive cause the overshoot 1703. The overshoot 1703 causes hyperpnea with the undershoot 1704 as a result. Then the cycle is repeated by the occurrence of another airflow limitation 1705, followed by another overshoot 1706 and another undershoot 1707. The ventilation time period 1708 is the time between the ventilation overshoot 1703 and the ventilation undershoot 1704. The inter-event time 1709 is the time between two consecutive airflow limitations, i.e. airflow limitation 1702 and airflow limitation 1705.

In the eighth embodiment, the processor 1400 is configured to receive respiratory information 1402 representative of respiration of the subject 504, and to determine a presence of a plurality of airflow limitations 1702, 1705 based on the respiratory information 1402. The processor 1400 is configured to determine, for each of the airflow limitations, the ventilation overshoot 1703, the ventilation undershoot 1704 or the ventilation time period 1708 between the ventilation overshoot 1703 and the ventilation undershoot 1704. The processor 1400 is configured to determine a trend based on the ventilation overshoot 1703, the ventilation undershoot 1704 or the ventilation time period 1708 for each of the plurality of airflow limitations. The processor 1400 is configured to determine the trait information based on the trend.

In a nineth embodiment, the processor 1400 is configured to receive sleep stage information 1408 representative of Rapid Eye Movement (REM) sleep and non-REM sleep during sleep of the subject 504. The processor 1400 is configured to determine a change in a difference between a first loop gain metric and a second loop gain metric during the sleep of the subject 504. The first loop gain metric is representative of an amount of loop gain during the REM sleep. The second loop gain metric is representative of an amount of loop gain during the non-REM sleep. The processor 1400 is configured to determine the trait information based on the change in the difference.

For example, the loop gain metric is determined based on the information as depicted in FIG. 18. FIG. 18 depicts in the bottom part a graph depicting the ventilation versus time while the subject 504 is using the respiratory support device 500 with a pressure as represented in the top part of the figure. From time 0 to ti, the subject 504 is breathing with a steady state ventilation at a eupneic level as indicated with line 1800. At ti, the pressure of the pressurized airflow 502 of the respiratory support device 500 is reduced, in this case from 10 to 4 cmHzO. As a result of the reduced pressure, the ventilation reduces to a minimum value. The ventilation increases till the ventilation reaches a new stable rate at t₂. The new stable rate has a difference 1802 with the eupneic level 1800. As a result of the difference 1802 with the eupneic level 1800, the chemical drive builds up. At t₃, the pressure of the pressurized airflow 502 is increased back to the original pressure. As a result, the ventilation increases and causes an overshoot 1804 relative to the eupneic level 1800. The ratio between the difference 1802 and the overshoot 1804 is a measure for the loop gain. More information about determining loop gain metrics is found in the study by Wellman, et.al, 2011. "A Method for Measuring and Modeling the Physiological Traits Causing Obstructive Sleep Apnea." Journal of Applied Physiology 110 (6): 1627-37. https://doi.org/10.1152/japplphysiol.00972.2010.

For example, the sleep stages are classified into two sleep stage classes, i.e., a REM sleep stage class and a non-REM sleep stage class. During the REM sleep stage class, the subject 504 is asleep and has Rapid Eye Movements, whereas during the non-REM sleep stage class, the subject 504 is asleep without Rapid Eye Movements. For example, the sleep stages are classified into three classes. The three classes are a wake sleep stage class, a REM sleep stage class, and a non-REM sleep stage class. During the wake sleep stage class, the subject 504 is awake and thus not sleeping. For example, the sleep stages are classified into four classes. The four classes are a wake sleep stage class, a REM sleep stage class, a light sleep stage class, and a deep sleep stage class. The light sleep stage class and the deep sleep stage class are non-REM sleep stage classes. For example, the sleep stages are classified into the classes N1, N2, N3, REM and Wake.

The sleep stage information 1408 is, for example, generated based on neurological signals of the subject 504. For example, the neurological signals are obtained via polysomnography (PSG), via electroencephalography (EEG), via electrooculography (EOG), via electromyography (EMG) or any combination of these. A sensor adapted to generate a sensor signal based on the neurological signals is, for example, mounted on a wearable device for the head or face, such as a headband. The sleep stage information 1408 is based on the neurological signals via use of an automated sleep stage classifier or via manual annotation. The output from the sleep stage classifier or from the manual annotation is provided to the processor 1400 as the sleep stage information 1408.

In addition or alternatively to using neurological signals, the sleep stage information 1408 is, for example, generated based on a surrogate measure of sleep. For example, the sleep stage information 1408 is based on respiratory information as described in US patent US11484256B2, hereby incorporated by reference. For example, the surrogate measure of sleep is based on changes in autonomic nervous system activity of the subject 504. A change in the autonomic nervous system activity is, for example, detected based on cardiac signals obtained with an appropriate sensor such as a reflective photoplethysmography (PPG) sensor, a transmissive PPG sensor or a remote PPG sensor, a ballistocardiographic sensor, or a seismocardiographic sensor. The reflective PPG sensor is, for example, arranged on the wrist or the face of the subject 504. The transmissive PPG sensor is, for example, arranged on the finger of the subject 504. The remote PPG sensor comprises, for example, an infrared camera. The ballistocardiographic sensor comprises, for example, an accelerometer or gyroscope attached to the body of the subject 504, or for example a pressure sensor mounted in the mattress or bed of the subject 504. The seismocardiographic sensor comprises, for example, an accelerometer mounted on the chest of the subject 504. The signals obtained by one or more of these sensors are, for example, used as input to a machine learning model trained to infer sleep stages. The input is, for example, based on manually crafted features correlating with sleep stages, such as a feature describing heart rate variability, or a feature of a time series describing heart rate progression during sleep (e.g. instantaneous heart rate). The input is, for example, input as raw data to the machine learning model.

In addition or alternatively to determining the sleep stage information 1408 as mentioned above, the sleep stage information 1408 is, for example, based on respiratory activity. Respiratory activity of the subject 504 is indicative of changes in autonomic nervous system activity associated with different sleep stages. Respiratory activity is, for example, measured with a sensor adapted to measure airflow or adapted to measure chest movements. For example, a sensor adapted to measure airflow comprises an oral cannula, a nasal cannula and/or a thermistor. For example, a sensor adapted to measure chest movements comprises a respiratory inductance plethysmography belt to be worn around the thorax of the abdomen. For example, information about the respiratory activity is provided by the respiratory information 1402. For example, the sensor adapted to measure respiratory activity comprises a pressure sensor mounted on the bed or mattress. For example, the sensor adapted to measure respiratory activity comprises a Doppler radar positioned near the subject 504. For example, the sensor for measuring respiratory activity comprises an accelerometer or a gyroscope mounted on the thorax, the abdomen and/or sternum of the subject 504. For example, the body position sensor 800 comprises an accelerometer and is used to measure the respiratory activity.

The respiratory event information is, for example, information associated with sleep disordered breathing (SDB) and/or OSA. For example, the respiratory example is based on a combination of airflow, respiratory effort and oxygen saturation. For example, the airflow is detected using an airflow sensor or a pressure sensor. For example, the respiratory effort is detected using an accelerometer arranged at the chest or abdomen of the subject 504. For example, the oxygen saturation is detected using a PPG sensor. For example, the respiratory event information is based on surrogate measurements, such as based on cardiac changes and/or respiratory changes. For example, the sensor that detects cardiac signals to detect changes in the autonomic nervous system activity is used to generate at least part of the respiratory event information. For example, the sensor that detects respiratory activity is used to generate at least part of the respiratory event information.

In a tenth embodiment, the processor 1400 is configured to receive respiratory information 1402 representative of respiration of the subject 504. The processor 1400 is configured to determine a presence of a plurality of SDB events based on the respiratory information 1402. The processor 1400 is configured to determine an inter-event time 1709 between two consecutive SDB events of the plurality of SDB events. The processor 1400 is configured to determine the trait information based on the inter-event time 1709. The inter-event time 1709 is depicted in FIG. 17.

### LOW MUSCLE RESPONSIVENESS

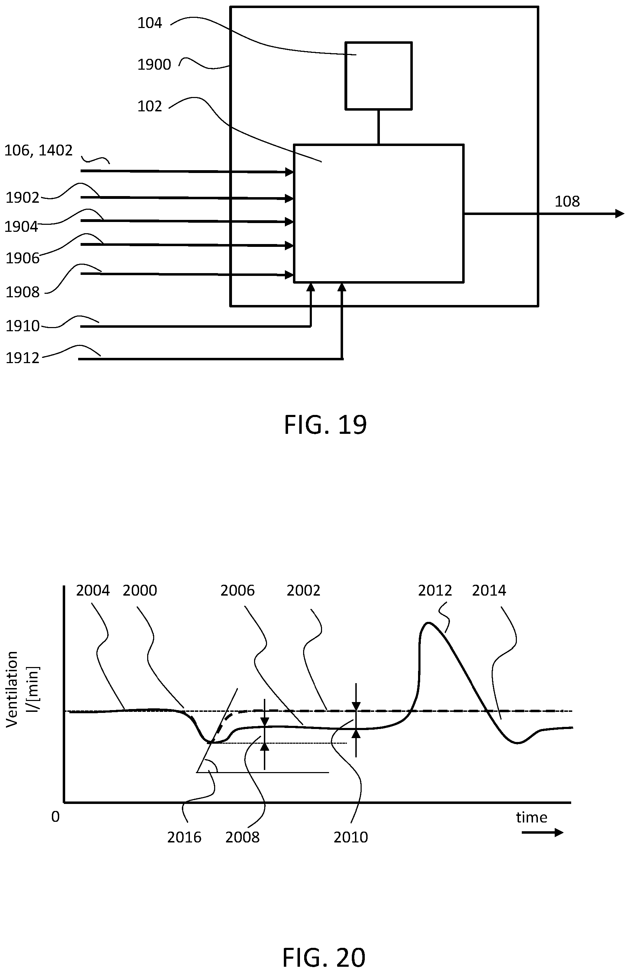

FIG. 19 depicts the processor 1900 according to a fourth aspect of the invention. The processor 1900 comprises a processing unit 102 and a memory 104 connected to the processing unit 102. The memory 104 is a computer-readable storage medium that stores a computer program product. The computer program product comprises instructions which, when executed by the processor 1900, cause a method according to the fourth aspect to be carried out. The fourth aspect is the same as the first aspect or as the second aspect or as the third aspect, except for the following. For example, the processor 1900 is the same as the processor 100, except for the following.

In an embodiment, the invention according to the fourth aspect is combined with any of the embodiments according to the first aspect, the second aspect or the third aspect of the invention.

According to the fourth aspect, the pathophysiological trait comprises a low muscle responsiveness 903 of muscles in an upper airway of the subject 504.

The processor 1900 is adapted to receive one or more of respiratory information 1402, EMG information 1902, heart rate information 1904, blood pressure information 1906, arousal information 1908, skin conductivity information 1910, and/or body temperature information 1912.

FIG. 20 depicts a first embodiment of the fourth aspect. In the first embodiment, the method comprises receiving the respiratory information 1402 representative of respiration of the subject 504. The respiratory information 1402 relates to ventilation. The method comprises determining an occurrence of an airflow limitation 2000 based on the respiratory information 1402. The method comprises determining a baseline ventilation 2004 before the airflow limitation 2000 based on the respiratory information 1402. The method comprises determining a recovery ventilation 2006 after the airflow limitation 2000 based on the respiratory information 1402. The method comprises determining the influence of the medication on the low muscle responsiveness based on a difference 2010 between the baseline ventilation 2004 and the recovery ventilation 2006.

In case of a subject 504 without low muscle responsiveness, the airflow limitation 2000 would cause the ventilation to decrease and then the ventilation would recover as indicated with line 2002. The ventilation then returns quickly back to the baseline ventilation 2004. However, in case of a subject 504 with low muscle responsiveness, the ventilation does not recover directly back to the baseline ventilation 2004. Instead, the ventilation recovers as indicated by line 2006. The recovery ventilation 2006 has increased compared to the ventilation during the start of the airflow limitation with difference 2008. However, the recovery ventilation 2006 is below the baseline ventilation 2004 with difference 2010. Because the recovery ventilation 2006 is below the baseline ventilation 2004, the chemical ventilatory drive increases over time, causing an overshoot 2012 in ventilation. This overshoot 2012 may cause another airflow limitation 2014 later on. In case the medication has a high influence on the muscle responsiveness, the difference 2008 will be higher and the difference 2010 will be lower than in case the medication has a low influence on the muscle responsiveness.

In a second embodiment, the method comprises receiving the electromyography (EMG) information 1902 representative of electrical activity of the muscles in the upper airway. The method comprises determining the influence of the medication on the low muscle responsiveness based on the EMG information 1902.

In a third embodiment, the medication comprises a norepinephrine reuptake inhibitor (NRI). The method comprises receiving heart rate information 1904 and/or receiving blood pressure information 1906. The method comprises determining the influence of the medication on the low muscle responsiveness based on the heart rate information 1904 and the blood pressure information 1906.

In a fourth embodiment, the medication comprises atomoxetine. The method comprises receiving heart rate information 1904 of the subject 504, and receiving arousal information 1908 of the subject 504. The method comprises determining a heart rate of the subject 504 in response to an arousal based on the heart rate information 1904 and the arousal information 1908. The method comprises determining the influence of the medication on the low muscle responsiveness based on the heart rate in response to the arousal.

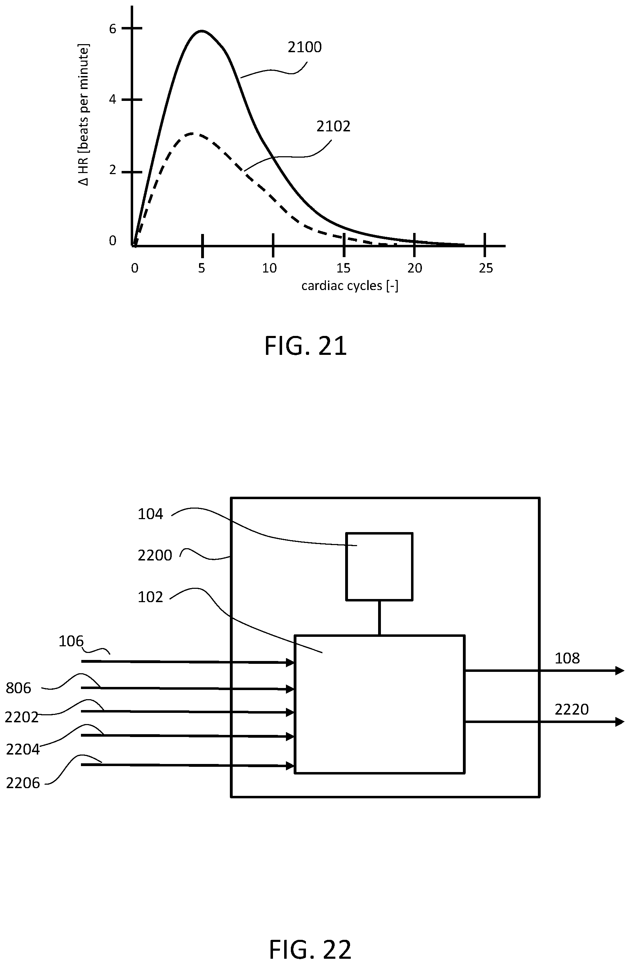

FIG. 21 depicts a heart rate according to the fourth embodiment of the fourth aspect. Line 2100 indicates a heart rate in response to an arousal in case the atomoxetine has a large influence on the subject. Atomoxetine increases muscle responsiveness. As a side effect, atomoxetine increases heart rate. The heart rate of line 2100 increases with about 6 beats per minute at five cardiac cycles after the arousal. Then, the heart rate starts to recover back to the heart rate as before the arousal. Line 2102 indicates a heart rate of the subject 504 in response to an arousal in case the atomoxetine has a low influence on the subject or no influence on the subject. The heart rate does not increase as much in response to an arousal as compared to line 2102. The heart rate response is blunted compared to line 2100. The heart rate of line 2102 only increases with 3 beats per minute at five cardiac cycles after the arousal. Then the heart rate starts to recover back to the heart rate as before the arousal. So, if the heart rate information 1904 indicates that the heart rate does not increase as much as earlier in the sleep session, this indicates that the influence of the atomoxetine is decreasing.

In an embodiment, the medication comprises oxybutynin. The method comprises receiving electrical skin conductivity information 1910. The method comprises determining the influence of the medication on the low muscle responsiveness based on the electrical skin conductivity information 1910.

In an embodiment, the medication comprises oxybutynin. The method comprises receiving body temperature information 1912. The method comprises determining the influence of the medication on the low muscle responsiveness based on the body temperature information 1912.

### LOW AROUSAL THRESHOLD

FIG. 22 depicts the processor 2200 according to a fifth aspect of the invention. The processor 2200 comprises a processing unit 102 and a memory 104 connected to the processing unit 102. The memory 104 is a computer-readable storage medium that stores a computer program product. The computer program product comprises instructions which, when executed by the processor 2200, cause a method according to the fifth aspect to be carried out. The fifth aspect is the same as the first aspect, the second aspect, the third aspect or the fourth aspect, except for the following. For example, the processor 2200 is the same as processor 100, except for the following.

The processor 2200 is configured to receive the positional signal 806, sleep architecture information 2202, a stimulus response signal 2204, and/or sleep spindle information 2206. The processor 2200 is configured to generate a stimulation signal 2220.

In an embodiment, the invention according to the fifth aspect is combined with any of the embodiments according to the first aspect, the second aspect, the third aspect or the fourth aspect of the invention.

In the fifth aspect, the pathophysiological trait comprises a low arousal threshold.

In a first embodiment, the medication comprises a hypnotic or a sedative.

In a second embodiment, the method comprises determining an influence of the medication on the low arousal threshold for a first time period 210 and a second time period 220. During the first time period 210 the influence of the medication on the low arousal threshold is larger than during the second time period 220. The method comprises determining the setting to provide the SDB therapy at a higher intensity level during the first time period than during the second time period.

In a third embodiment, the method comprises determining an influence of the medication on the low arousal threshold for a first time period 210 and a second time period 220. During the first time period 210 the influence of the medication on the low arousal threshold is larger than during the second time period 220. The method comprises determining whether the subject 504 is in a supine position. The method comprises generating the stimulation signal 808 when the subject 504 is in the supine position. The stimulation signal 808 is adapted to cause the stimulator 802 to provide a stimulus to the subject 504 to prompt the subject 504 to change to a body position different than the supine position. The stimulus is provided at a higher intensity level during the first time period than during the second time period.

The processor 2200 is adapted to receive the positional signal 806 from the body position sensor 800 of the positional therapy device 700 of FIG. 8. The positional therapy device 700 of FIG. 8 comprises the stimulator 802 to provide the stimulus in case the subject 504 is in the supine position. For example, a vibration generated by the stimulator 802 has a larger amplitude and/or duration during the first time period 210 than during the second time period 220. The processor 2200 is adapted to receive the setting 108 and to adjust the stimulation signal 808 accordingly.

In a fourth embodiment, the method comprises receiving sleep architecture information 2202 representative of a first sleep architecture and a second sleep architecture of the subject 504. The first sleep architecture is during a first time period 210. The second sleep architecture is during a second time period 220.

The method comprises determining a change between the first sleep architecture and the second sleep architecture based on the sleep architecture information 2202. The method comprises determining the influence of the medication on the low arousal threshold based on the change between the first sleep architecture and the second sleep architecture.

In a fifth embodiment, the medication comprises an orexin receptor antagonist. The change between the first sleep architecture and the second sleep architecture comprises a change in deep sleep and/or a change in REM sleep.

In a sixth embodiment, the method comprises generating the stimulation signal 2220. The stimulation signal 2220 is adapted to cause a stimulator to provide a stimulus to the subject 504. For example, the stimulator comprises a light generator, a sound generator, or a vibration generator, or a pulsed air generator. The method comprises receiving the stimulus response signal 2204 representative of a response of the subject 504 to the stimulus. The method comprises determining the influence of the medication on the low arousal threshold based on the stimulus response signal 2204. For example, the stimulus response signal 2204 is representative of a cardiac parameter, a neurological parameter or a respiratory parameter or a body movement parameter.

For example, the stimulator comprises a light generator, a sound generator, or a vibration generator, or a pulsed air generator.

In a seventh embodiment, the method comprises receiving sleep spindle information 2206 representative of occurrences of sleep spindles of the subject 504. The method comprises determining the influence of the medication on the low arousal threshold based on the sleep spindle information 2206.

In an eighth embodiment, the method comprises receiving ventilatory information representative of ventilation of the subject during the sleep session. The method comprises receiving arousal information corresponding to the occurrence of at least two arousals during the sleep session. The method comprises determining a level of ventilation for each occurrence of the at least two arousals based on the ventilatory information and the arousal information. The method comprises comparing the levels of ventilation with each other. The method comprises determining the trait information based on comparing the levels of ventilation with each other.

### CHANGES IN DOSAGE

The sixth aspect of the invention relates to changes on dosage of the medication. The embodiments of the sixth aspect can be combined with any one of the embodiments of the first, second, third, fourth, and fifth aspect of the invention. The embodiments of the sixth aspect can be combined with any one of the four pathophysiological traits as described above. The embodiments of the sixth aspect can be combined with any one of the three therapy devices as described above.

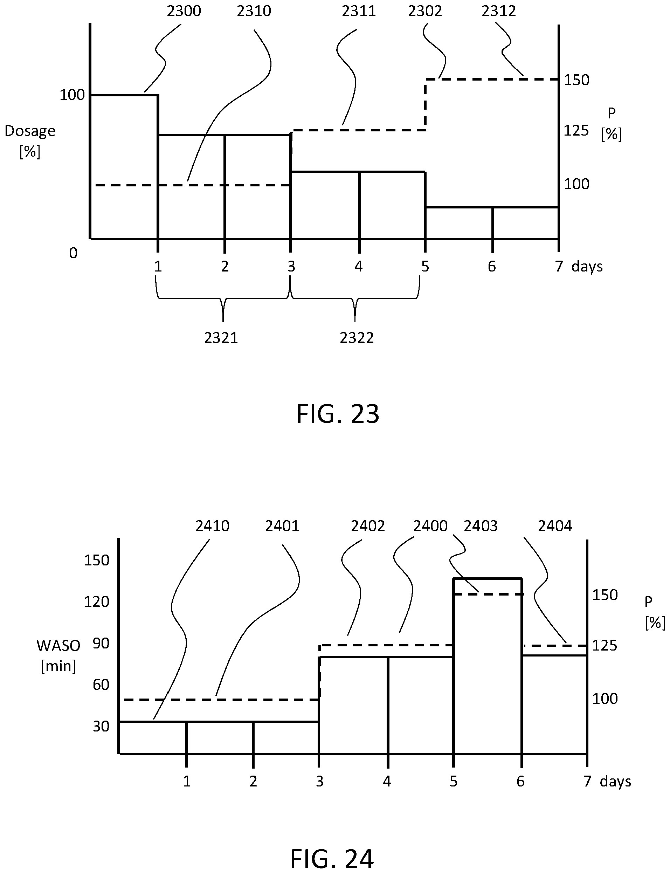

FIG. 23 depicts a first embodiment of the sixth aspect. The x-axis shows days 0-7. The bars 2300 show the dosage of the medication for each day. The dosage at day 0 is set at 100%. The dosages on the other days are related to the dosage on day 0. The line 2302 shows the pressure of the pressurized airflow 502 provided by the respiratory support device 500 for each day. The pressure on day 0 is set at 100%. The pressures on the other days are related to the pressure at day 0. Line 2310 shows a pressure of 100%. Line 2311 shows a pressure of 125%. Line 2312 shows a pressure of 150%.

In the first embodiment of the medication information 106 is representative of a dosage during a first dosage period 2321 and a dosage during a second dosage period 2322.

The method comprises determining the setting 108 of the therapy device to provide the SDB therapy having a therapy parameter. The therapy parameter has a first value during the first dosage period 2321 and has a second value during the second dosage period 2322. The first value is different from the second value. In this embodiment, the therapy parameter is the pressure of the pressurized airflow 502 provided by the respiratory support device 500. The first value is 100% as indicated by line 2310, whereas the second value is 125% as indicated by line 2311.

FIG. 23 shows that the dosage is reduced from 100% on day 0 to 75% on day 1, whereas the pressure remains 100% till day 3. Because of the concentration of the medication, the medication still provides sufficient influence on the pathophysiological trait on days 1 and 2 even though the dosage has reduced.

The medication information 106 comprises a dosage reduction scheme during a dosage reduction period. The dosage reduction period includes the first dosage period 2321 and the second dosage period 2322. The dosage reduction scheme is representative of a dosage reduction from the first dosage of 100% to the second dosage of 25% over the dosage reduction period. The method comprises determining the setting 108 of the therapy device to adjust the therapy parameter during the dosage reduction period based on the dosage reduction scheme.

FIG. 23 shows how the dosage is reduced from 100% on day 0 to 25% on day 7 according to the dosage reduction scheme. The dosage reduction period includes days 1-7. The pressure is adjusted based on the dosage reduction scheme.

FIG. 24 depicts a second embodiment of the sixth aspect. In the second embodiment, the method comprises receiving sleep information representative of a sleep parameter during the dosage reduction period or the second dosage period 2322. The sleep parameter is representative of a sleep quality of the subject 504. The method comprises determining, based on the sleep information, whether the sleep parameter is indicative of a deterioration of the sleep quality. The method comprises in response to determining the deterioration of the sleep quality, determining the setting 108 of the therapy device to adjust the therapy parameter.

FIG. 24 depicts on the x-axis days 0-7. Line 2400 indicates the therapy parameter, in this case the pressure of the pressurized airflow 502 of the respiratory support device 500. The pressure at day 0 is set at 100%. The pressures on the other days are related to the pressure at day 0. The bars 2410 indicate the Wake After Sleep Onset (WASO) in minutes, which represents the amount of time the subject 504 is awake during a sleep session after initially fallen asleep. The WASO is a measure of sleep quality. For good sleep quality, the WASO should be about 30 minutes or less.

From days 0-3, the pressure is at 100%, which results in a WASO of about 30 minutes. On day 3, the pressure is increased to 125%, because of the reduced influence of the medication on the pathophysiological trait. As a result of the increased pressure, the WASO worsens to about 80 minutes. This indicates that the sleep quality has deteriorated. On day 5, the pressure is further increased to 150%, because of the further reduced influence of the medication on the pathophysiological trait. However, this results in an excessively large WASO of about 120 minutes. The large WASO indicates that the sleep quality is unacceptably low. Therefore, the pressure is reduced on day 6 to 125%. With the pressure at 125%, a balance is achieved between proper SDB therapy and an acceptable sleep quality.

For example, the sleep parameter comprises one of a user input, sleep stage information 1408, sleep architecture information 2202, sleep quality, and/or sleep time misperception. For example, the sleep parameter representative of a sleep quality is based on wake periods during the sleep session, such as sleep onset latency (SOL), the number and durations of awakenings, the number and durations of long awakenings, such as awakenings longer than 5 minutes.

### TOLERANCE

The seventh aspect of the invention relates to tolerance to the medication. The embodiments of the seventh aspect can be combined with any one of the embodiments of the first, second, third, fourth, fifth, and sixth aspect of the invention. The embodiments of the seventh aspect can be combined with any one of the four pathophysiological traits as described above. The embodiments of the seventh aspect can be combined with any one of the three therapy devices as described above.

In an embodiment, the method comprises: receiving patient information relating to a tolerance of the medication; determining a tolerance to the medication based on the patient information; and determining the setting of the therapy device based on the tolerance to the medication.

In an embodiment, the method comprises receiving comorbidity information representative of a drug metabolism of the subject affected by a comorbidity of the subject. The method comprises determining a tolerance to the medication based on the comorbidity information. The method comprises determining the setting of the therapy device based on the tolerance to the medication.

In an embodiment, the method comprises receiving drug-drug interaction information representative of a drug metabolism of the subject affected by an interaction of the medication and a further medication used by the subject. The method comprises determining a tolerance to the medication based on the drug-drug interaction information. The method comprises determining the setting of the therapy device based on the tolerance to the medication.

In an embodiment, the method comprises receiving further medication information representative of further medication used by the subject. The further medication does not influence the pathophysiological trait of the subject. The further medication information comprises information of a tolerance to the further medication. The method comprises determining a tolerance to the medication based on the information of a tolerance to the further medication. The method comprises determining the setting of the therapy device to adjust the intensity level of the SDB therapy based on the tolerance to the medication.

In an embodiment, the further medication information comprises information of the tolerance of the subject to the further medication.

In an embodiment, the further medication information comprises information of the tolerance of a group of subjects to the further medication.

In an embodiment, the medication and the further medication are in the same category.

### MEDICATION INTAKE

The eighth aspect of the invention relates to medication intake. The embodiments of the seventh aspect can be combined with any one of the embodiments of the first, second, third, fourth, fifth, sixth, and seventh aspect of the invention. The embodiments of the eighth aspect can be combined with any one of the four pathophysiological traits as described above. The embodiments of the eighth aspect can be combined with any one of the three therapy devices as described above.

For example, the medication information comprises medication intake information representative of a medication intake of the subject. The method comprises determining the setting based on the medication intake information.

Whether, and/or when the subject takes the medication may affect the influence of the medication on the pathophysiological trait. For example, if the subject forgets to take the medication or takes the medication at the wrong time, the medication may have less influence on the pathophysiological trait during the sleep session. So by taking the medication intake information into account, the therapy device provides improved SDB therapy to the subject.

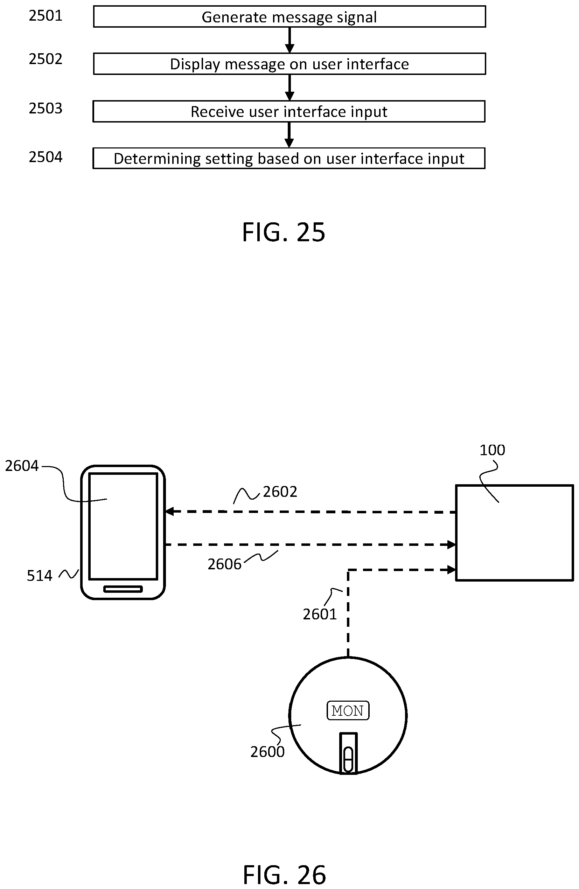

FIG. 25 depicts a first embodiment according to the eighth aspect. The method comprises at 2501 generating a message signal adapted to display on a user interface a message relating to a request for the medication intake information. At 2502, the message is displayed on the user interface. The method comprises at 2503 receiving a user interface input via the user interface in response to the message. The method comprises at 2504 determining the setting based on the user interface input.

FIG. 26 depicts a second embodiment according to the eighth aspect. The method comprises receiving with the processor 100 from a medication dispenser 2600 medication intake information 2601. The medication dispenser 2600 is adapted to release the medication as prescribed. When the medication dispenser 2600 releases the medication, for example a pill, the medication dispenser 2600 sends correspondingly the medication intake information 2601 to the processor 100.

Optionally in this embodiment, the processor 100 generates a message signal 2602 to display on a user interface, e.g., mobile device 514, a message 2604 relating to a request for the medication intake information. The mobile device 514 generates the user interface input signal 2606 representative of the user interface input given by the subject in response to the message. The processor 100 determines the setting based on the user interface input and/or the medication information 2601 from the medication dispenser 2600.

Optionally, the processor 100 detects whether the medication intake information from the medication dispenser is inconsistent with the medication intake information according to the user interface input signal 2606 from the user interface. The processor 100 generates an alarm signal in case the medication intake information from the medication dispenser is inconsistent with the medication intake information from the user interface input.

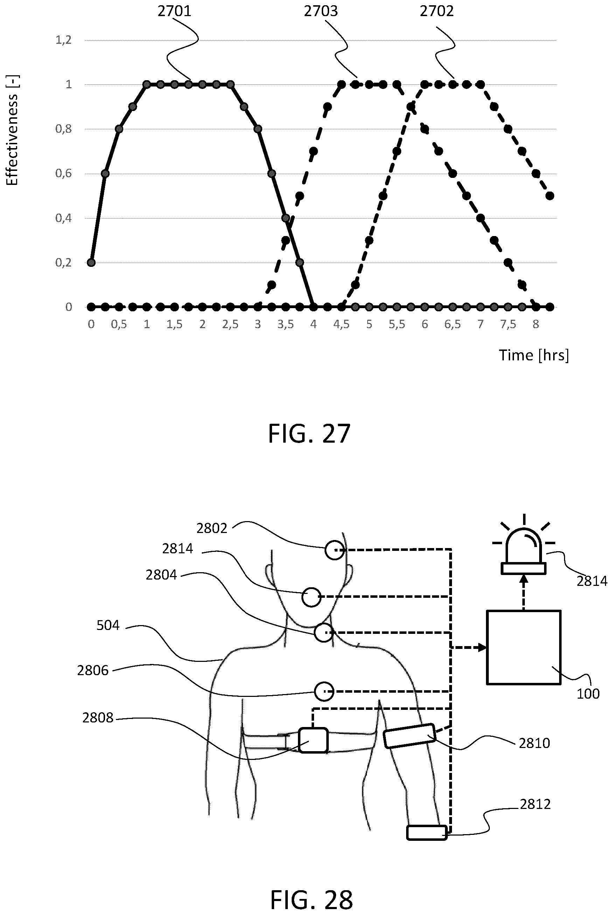

FIG. 27 depicts a third embodiment according to the eighth aspect. The medication information is indicative of a first medication and a second medication used by the subject. The method comprises determining a first setting of the therapy device based on the trait information and the medication information of the first medication. The method comprises determining a second setting of the therapy device based on the trait information and the medication information of the second medication. The method comprises determining the setting of the therapy device based on the first setting and the second setting.

The first medication has an effectiveness as depicted with line 2701. The first medication reaches the maximum effectiveness at time = 1 of the sleep session. After about time =2.5 hours, the effectiveness reduces till the first medication is no longer effective at time =4 hours. The first medication has an effectiveness as depicted with line 2701. The second medication starts to become effective after time t=4.5 hours, and reaches the maximum effectiveness at time = 6 of the sleep session. The second medication remains effective till the end of the sleep session at time = 8 hours.

Around time = 4 hours, neither the first medication nor the second medication has a high effectiveness. As a result, the setting of the therapy around time = 4 hours is set to provide therapy with a high intensity. The high intensity therapy may be provided by providing a third medication instead of the second medication or in addition to the second medication. The third medication has an effectiveness as depicted with line 2703. The effectiveness is the same as for the second medication, except that the effectiveness of the third medication starts earlier, at time = 3 hours. As a result, the setting of the therapy around time = 4 hours can be reduced and still provide proper SDB therapy.

### INFORMATION FROM THE SUBJECT

The embodiments described above make use of information. Some of that information is obtained via measurements of physiological parameters of the subject 504. This section indicates examples of how various information is obtained via measurements on the subject 504.

FIG. 28 depicts obtaining information from the subject 504 for use in any one of the aspects of the invention as described above. The information is provided to the processor 100.

For example, an EEG measurement is performed on the subject 504. For an EEG measurement, multiple EEG electrodes 2802 are arranged on the scalp of the subject 504. The EEG electrodes 2802 generate an EEG signal representative of brain activity of the subject 504. The EEG signal provides, for example, the sleep stage information 1408 for the embodiment of FIG. 14, the arousal information 1908 of FIG. 19, and/or the sleep spindle information 2206 of FIG. 22.

For example, an EMG measurement is performed on the subject 504. For an EMG measurement, one or more EMG electrodes 2804 are arranged at the throat area of the subject 504. The EMG electrodes 2804 generate an EMG signal representative of muscle activity of the muscles near the throat. This muscle activity represents muscle activity of the muscles of the upper airway. The EMG signal provides, for example, the EMG information 1902 of FIG. 19.

For example, an ECG measurement is performed on the subject 504. For an ECG measurement, one or more ECG electrodes 2806 are arranged at the heart area of the subject 504. The ECG electrodes 2806 generate an ECG signal representative of cardiac activity of the heart. The ECG signal provides, for example, the sleep stage information 1408 of FIG. 14, and/or the heart rate information 1904 of FIG. 19.

For example, an accelerometer 2808 is attached to the chest area of the subject 504. The accelerometer 2808 provides measurements of movements and/or vibrations of the subject 504. The accelerometer 2808 generates an acceleration signal based on the movements and/or vibrations of the subject 504. The acceleration signal provides, for example, the body position signal 806 of FIG. 8, and/or the heart rate information 1904 of FIG. 19. As the acceleration signal provides heart rate information 1904, the sleep stage information 1408 of FIG. 14 and/or the arousal information 1908 of FIG. 19 can be determined based on the acceleration signal.

For example, a blood pressure cuff 2810 is attached to the arm of the subject 504. The blood pressure cuff 2810 provides a blood pressure signal representative of a blood pressure or a change of blood pressure. The blood pressure signal provides, for example, the blood pressure information 1906 of FIG 10 or FIG. 19.

For example, a PPG measurement is performed on the subject 504, for example on the wrist of the subject 504. The PPG device 2812 performing the PPG measurement provides a PPG signal representative of blood volume changes. The PPG signal provides, for example, the blood pressure information 1906 of FIG 10 or FIG. 19, the oxygen saturation information 1406 of FIG. 14, the heart rate information 1904 of FIG. 19. As the PPG signal provides heart rate information 1904, the sleep stage information 1408 of FIG. 14 and/or the arousal information 1908 of FIG. 19 can be determined based on the acceleration signal.

For example, an airflow measurement is performed on the subject 504 with an airflow sensor 2810. The airflow sensor 2814 generates a signal representative of an airflow inhaled and/or exhaled by the subject. For example, the airflow sensor 2814 generates a signal representative of ventilation of the subject 504. The airflow sensor 2814 provides, for example, the respiratory information 1402.

For example, one or more of the sensors depicted in FIG. 28 are arranged in the patient interface 510 of the respiratory support device 500. For example, the airflow sensor 2814, the PPG device 2812, the EMG electrodes 2804, and/or the accelerometer 2808 are arranged in or on the patient interface 510.

Further, FIG. 28 depicts the stimulator 2814 to provide a stimulus to the subject 504 in response to the stimulation signal 2220.

### FURTHER STATEMENTS

It will be understood that the disclosed methods are computer-implemented methods. As such, there is also proposed a concept of a computer program comprising code means for implementing any described method when said program is run on a processing system.

The skilled person would be readily capable of developing processor 100 for carrying out any herein described method. Thus, each step of a flow chart may represent a different action performed by the processor 100, and may be performed by a respective module of the processor.

As discussed above, the processor 100 performs data processing. The processor 100 can be implemented in numerous ways, with software and/or hardware, to perform the various functions required. The processor 100 employs, for example, one or more microprocessors programmed using software (e.g. microcode) to perform the required functions. The processor 100 may be implemented as a combination of dedicated hardware to perform some functions and one or more programmed microprocessors and associated circuitry to perform other functions.

For example, the processor 100 or the processing unit 102 comprise one or more of a conventional microprocessor, an application specific integrated circuit (ASIC), or a field-programmable gate array (FPGA). Thus, the processor may be embodied as a digital and/or analog processing system.

For example, the memory 104 comprises volatile computer memory 104 and/or nonvolatile computer memory 104 such as RAM, PROM, EPROM, and EEPROM. The memory 104 may be encoded with one or more programs that, when executed on one or more processors, perform the required functions. The memory 104 comprises, for example, various storage media fixed within the processor or transportable to the processor, such that the one or more programs stored thereon can be loaded into the processor.

Functions implemented by a processor 100 may be implemented by a single processor or by multiple separate processing units which may together be considered to constitute a "processor". Such processing units may in some cases be remote from each other and communicate with each other in a wired or wireless manner.

The memory 104 is, for example, an optical storage medium or a solid-state medium supplied together with or as part of other hardware, but may also be distributed in other forms, such as via the Internet or other wired or wireless telecommunication systems.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

### CLAUSES

Embodiments of the invention are presented in the following clauses.
Clause 1. A computer-implemented method for determining a setting 108 of a therapy device adapted to provide sleep disordered breathing (SDB) therapy to a subject 504, the method comprising: receiving medication information 106 representative of medication used by the subject 504; determining trait information based on the medication information 106, wherein the trait information is representative of an influence of the medication on a pathophysiological trait of the subject 504, wherein the pathophysiological trait has an influence on the SDB of the subject 504; and determining the setting 108 of the therapy device based on the trait information.
Clause 2. The method according to clause 1, wherein the medication information 106 comprises information about a half-life of the medication, or a dosage of the medication, or a tolerance to the medication, or an effectiveness of the medication, or a duration of use of the medication, or a pharmacokinetic (PK) model, or a pharmacodynamic (PD) model, or a pharmacokinetic/ pharmacodynamic (PK/PD) model.
Clause 3. The method according to any one of the preceding clauses, comprising determining trait information during a first time period and during a second time period, wherein during the first time period the influence of the medication on the pathophysiological trait is larger than during the second time period; determining the setting 108 of the therapy device to provide the SDB therapy having a therapy parameter, wherein the therapy parameter has a first value during the first time period and has a second value during the second time period, wherein the first value is different from the second value.
Clause 4. The method according to clause 3, wherein the therapy parameter relates to at least one of: an intensity level of the SDB therapy; a rate of change of the intensity level of the SDB therapy; a range of the intensity level of the SDB therapy; or a difference in intensity level of the SDB therapy between inhalation and exhalation of the subject 504.
Clause 5. The method according to clause 3 or 4, adjusting the therapy device according to the setting 108 to provide the SDB therapy having the first value during the first time period and having the second value during the second time period.
Clause 6. The method according to any one of clauses 3-5, wherein the first time period and the second time period are in a same sleep session of the subject 504, or wherein the first time period and the second time period are in different sleep sessions of the subject 504.

### HIGH PHARYNGEAL COLLAPSIBILITY

Clause 7. The method according to any one of the preceding clauses, wherein the pathophysiological trait comprises a high pharyngeal collapsibility.
Clause 8. The method according to clause 7, wherein the medication comprises a diuretic, wherein the method comprises: estimating a fluid shift in the subject 504 during a sleep session caused by the diuretic based on the medication information 106 determining the trait information based on the fluid shift.
Clause 9. The method according to clause 8, comprising: estimating a urinary time period in the sleep session based on the medication information 106, wherein during the urinary time period the subject 504 has likely a need to urinate; determining the setting 108 of the therapy device to provide the SDB therapy at a lower intensity level during the urinary time period than before the urinary time period.
Clause 10. The method according to any one of clauses 8-9, comprising receiving blood pressure information 1906 representative of a blood pressure of the subject 504; determining the trait information based on the blood pressure information 1906.
Clause 11. The method according to any one of clauses 7-10, wherein the medication comprises a weight loss medication, wherein the method comprises providing a weight loss estimation based on the medication information 106; and determining the trait information based on the weight loss estimation.
Clause 12. The method according to any one of clauses 7-11, comprising: receiving neck circumference information or body weight information representative of a body weight of the subject 504; determining the trait information based on the neck circumference information or the body weight information.
Clause 13. The method according to any one of clauses 11-12, comprising: receiving AHI information representative of an Apnea Hypopnea Index (AHI) of the subject 504 during a sleep session without SDB therapy; determining the trait information based on the AHI information.
Clause 14. The method according to any one of clauses 7-13, wherein the medication comprises a nasal decongestant.

### HIGH LOOP GAIN

Clause 15. The method according to any one of the preceding clauses, wherein the pathophysiological trait comprises a high loop gain.
Clause 16. The method according to clause 15, wherein the therapy device is adapted to provide oxygen to the subject 504, wherein determining the setting 108 of the therapy device comprises determining a flow rate of the oxygen to the subject 504 based on the trait information.
Clause 17. The method according to clause 15, wherein the therapy device is adapted to provide carbon dioxide to the subject 504, wherein determining the setting 108 of the therapy device comprises determining a flow rate of the carbon dioxide to the subject 504 based on the trait information.
Clause 18. The method according to any one of clauses 15-17, comprising: receiving respiratory information 1402 representative of respiration of the subject 504 during a sleep session; determining a beginning of an airflow limitation based on the respiratory information 1402; determining whether the airflow limitation is still occurring after a maximum waiting time; in case the airflow limitation is still occurring after the maximum waiting time, generating a stimulation signal 2220, wherein the stimulation signal 2220 is adapted to cause a stimulator to provide a stimulus to the subject 504, wherein the stimulus is adapted to cause arousal of the subject 504.
Clause 19. The method according to clause 18, comprising receiving information about a eupneic ventilation of the subject 504; receiving respiratory information representative of respiration of the subject 504; determining the maximum waiting time by: detecting a plurality of airflow limitations based on the respiratory information 1402; waiting a waiting time after a start of each airflow limitation, wherein the waiting time is different for each airflow limitation; generating a stimulation signal 2220 when the waiting time expires, wherein the stimulation signal 2220 is adapted to cause a stimulator to provide a stimulus to the subject 504; receiving ventilation information representative of a ventilation of the subject 504 after the stimulus; comparing the ventilation information with the eupneic ventilation; and selecting as the maximum waiting time the waiting time corresponding to the ventilation of the subject 504 being different from the eupneic ventilation within a threshold.
Clause 20. The method according to clause 18 or 19, wherein the therapy device comprises a respiratory support device 500 adapted to provide pressurized airflow 502 to the subject 504, and wherein the therapy device comprises the stimulator, wherein the stimulation signal 2220 is adapted to cause the respiratory support device 500 to provide the pressurized airflow 502 to the subject 504 in a series of pulses.
Clause 21. The method according to any one of clauses 15-20, wherein the therapy device comprises a respiratory support device 500 adapted to provide pressurized airflow 502 to the subject 504, wherein the method comprises: determining the trait information during a first time period and during a second time period, wherein during the first time period the influence of the medication on the high loop gain is larger than during the second time period; determining the setting 108 the therapy device to provide the pressurized airflow 502 with an inspiration pressure and an expiration pressure, wherein a difference between the inspiration pressure and the expiration pressure is larger during the second time period than during the first time period.
Clause 22. The method according to any one of clauses 15-21, wherein the therapy device comprises a respiratory support device 500 adapted to provide pressurized airflow 502 to the subject 504, wherein the method comprises: receiving respiratory information 1402 representative of respiration of the subject 504 during a sleep session; determining an SDB event based on the respiratory information 1402; determining the setting 108 of the therapy device to provide the pressurized airflow 502 with an inspiration pressure and an expiration pressure, wherein a difference between the inspiration pressure and the expiration pressure is larger after the SDB event than before the SDB event.
Clause 23. The method according to any one of clauses 15-22, wherein the medication comprises a carbonic anhydrase enzyme inhibitor (CAI).
Clause 24. The method according to clause 23, comprising receiving oxygen saturation information 1406 representative of an oxygen saturation of the subject 504; determining the trait information based on the oxygen saturation information 1406.
Clause 25. The method according to clause 15-24, comprising: receiving respiratory information 1402 representative of respiration of the subject 504; determining a presence of a plurality of SDB events based on the respiratory information 1402; determining, for each of the plurality of SDB events, a ventilation overshoot 1703, a ventilation undershoot 1704 or a ventilation time period 1708 between the ventilation overshoot 1703 and the ventilation undershoot 1704; determining a trend based on the ventilation overshoot 1703, the ventilation undershoot 1704 or the ventilation time period 1708 for each of the plurality of SDB events; determining the trait information based on the trend.
Clause 26. The method according to clause 15-25, comprising: receiving sleep stage information 1408 representative of Rapid Eye Movement (REM) sleep and non-REM sleep during sleep of the subject 504; determining a change in a difference between a first loop gain metric and a second loop gain metric during the sleep of the subject 504, wherein the first loop gain metric is representative of an amount of loop gain during the REM sleep; wherein the second loop gain metric is representative of an amount of loop gain during the non-REM sleep; determining the trait information based on the change in the difference.
Clause 27. The method according to clause 15-26, comprising: receiving respiratory information 1402 representative of respiration of the subject 504; determining a presence of a plurality of SDB events based on the respiratory information 1402; determining an inter-event time 1709 between two consecutive SDB events of the plurality of SDB events; determining the trait information based on the inter-event time 1709.

### LOW MUSCLE RESPONSIVENESS

Clause 28. The method according to any one of the preceding clauses, wherein the pathophysiological trait comprises a low muscle responsiveness of muscles in an upper airway of the subject 504.
Clause 29. The method according to clause 27, comprising receiving respiratory information representative of respiration of the subject 504; determining an occurrence of an airflow limitation based on the respiratory information 1402; determining a baseline ventilation before the airflow limitation based on the respiratory information 1402; determining a recovery ventilation after the airflow limitation based on the respiratory information 1402; determining the influence of the medication on the low muscle responsiveness based on a difference between the baseline ventilation and the recovery ventilation.
Clause 30. The method according to clause 27 or 28, comprising receiving electromyography (EMG) information representative of electrical activity of the muscles in the upper airway, determining the influence of the medication on the low muscle responsiveness based on the EMG information 1902.
Clause 31. The method according to any one of clauses 27 to 29, wherein the medication comprises a norepinephrine reuptake inhibitor (NRI), wherein the method comprises: receiving heart rate information 1904; receiving blood pressure information 1906; determining the influence of the medication on the low muscle responsiveness based on the heart rate information 1904 and the blood pressure information 1906.
Clause 32. The method according to any one of clauses 27 to 30, wherein the medication comprises atomoxetine, wherein the method comprises: receiving heart rate information 1904 of the subject 504; receiving arousal information 1908 of the subject 504; determining a heart rate of the subject 504 in response to an arousal based on the heart rate information 1904 and the arousal information 1908; determining the influence of the medication on the low muscle responsiveness based on the heart rate in response to the arousal.
Clause 33. The method according to any one of clauses 28-32, wherein the medication comprises oxybutynin, wherein the method comprises: receiving electrical skin conductivity information 1910; determining the influence of the medication on the low muscle responsiveness based on the electrical skin conductivity information 1910.

### LOW AROUSAL THRESHOLD

Clause 34. The method according to any one of the preceding clauses, wherein the pathophysiological trait comprises a low arousal threshold.
Clause 35. The method according to clause 34, wherein the medication comprises a hypnotic or a sedative.
Clause 36. The method according to clause 34 or 35, comprising: determining an influence of the medication on the low arousal threshold for a first time period and a second time period, wherein during the first time period the influence of the medication on the low arousal threshold is larger than during the second time period; determining the setting to provide the SDB therapy at a higher intensity level during the first time period than during the second time period.
Clause 37. The method according to any one of clauses 34-36, comprising: determining an influence of the medication on the low arousal threshold for a first time period and a second time period, wherein during the first time period the influence of the medication on the low arousal threshold is larger than during the second time period; determining whether the subject 504 is in a supine position; generating a stimulation signal 2220 when the subject 504 is in the supine position, wherein the stimulation signal 2220 is adapted to cause a stimulator to provide a stimulus to the subject 504 to prompt the subject 504 to change to a body position different than the supine position, wherein the stimulus is provided at a higher intensity level during the first time period than during the second time period.
Clause 38. The method according to clause 33 or 34, comprising: receiving sleep architecture information 2202 representative of a first sleep architecture and a second sleep architecture of the subject 504, wherein the first sleep architecture is during a first time period, wherein the second sleep architecture is during a second time period; determining a change between the first sleep architecture and the second sleep architecture based on the sleep architecture information 2202; determining the influence of the medication on the low arousal threshold based on the change between the first sleep architecture and the second sleep architecture.
Clause 39. The method according to clause 38, wherein the medication comprises an orexin receptor antagonist, wherein the change between the first sleep architecture and the second sleep architecture comprises a change in deep sleep and/or a change in REM sleep.
Clause 40. The method according to any one of clauses 34-39, comprising: generating a stimulation signal 2220, wherein the stimulation signal 2220 is adapted to cause a stimulator to provide a stimulus to the subject 504; receiving a stimulus response signal 2204 representative of a response of the subject 504 to the stimulus; determining the influence of the medication on the low arousal threshold based on the stimulus response signal 2204.
Clause 41. The method according to clause 40, wherein the stimulator comprises a light generator, a sound generator, or a vibration generator, or a pulsed air generator.
Clause 42. The method according to clause 40 or 41, wherein the stimulus response signal 2204 is representative of a cardiac parameter, a neurological parameter or a respiratory parameter or a body movement parameter.
Clause 43. The method according to any one of clauses 34-42, comprising: receiving sleep spindle information 2206 representative of occurrences of sleep spindles of the subject 504; determining the influence of the medication on the low arousal threshold based on the sleep spindle information 2206.
Clause 44. The method according to any one of clauses 34-43, comprising: receiving ventilatory information representative of ventilation of the subject during the sleep session; receiving arousal information corresponding to the occurrence of at least two arousals during the sleep session; determining a level of ventilation for each occurrence of the at least two arousals based on the ventilatory information and the arousal information; comparing the levels of ventilation with each other; determining the trait information based on comparing the levels of ventilation with each other.

### CHANGES IN DOSAGE

Clause 45. The method according to any one of the preceding clauses, wherein the medication information 106 is representative of a first dosage during a first dosage period 2321 and a second dosage during a second dosage period 2322, wherein the first dosage is higher than the second dosage, wherein the method comprises determining the setting 108 of the therapy device to provide the SDB therapy having a therapy parameter, wherein the therapy parameter has a first value during the first dosage period 2321 and has a second value during the second dosage period 2322, wherein the first value is different from the second value.
Clause 46. The method according to clause 45, wherein the medication information 106 comprises a dosage reduction scheme during a dosage reduction period, wherein the dosage reduction period is between the first dosage period 2321 and the second dosage period 2322, wherein the dosage reduction scheme is representative of a dosage reduction from the first dosage to the second dosage over the dosage reduction period, wherein the method comprises determining the setting 108 of the therapy device to adjust the therapy parameter during the dosage reduction period based on the dosage reduction scheme.
Clause 47. The method according to clause 41 or 42, comprising: receiving sleep information representative of a sleep parameter during the dosage reduction period or the second dosage period 2322, wherein the sleep parameter is representative of a sleep quality of the subject 504; determining, based on the sleep information, whether the sleep parameter is indicative of a deterioration of the sleep quality; in response to determining the deterioration of the sleep quality, determining the setting 108 of the therapy device to adjust the therapy parameter.
Clause 48. The method according to clause 47, wherein the sleep parameter comprises one of: a user input; sleep stage information 1408; sleep architecture information 2202; sleep quality; sleep time misperception.

### TOLERANCE

Clause 49. The method according to any one of the preceding clauses, comprising: receiving patient information relating to a tolerance of the medication; determining a tolerance to the medication based on the patient information; determining the setting 108 of the therapy device based on the tolerance to the medication.
Clause 50. The method according to clause 49, comprising: wherein the patient information comprises comorbidity information representative of a drug metabolism of the subject 504 affected by a comorbidity of the subject 504; determining the tolerance to the medication based on the comorbidity information.
Clause 51. The method according to clause 49 or 50, comprising: wherein the patient information comprises drug-drug interaction information representative of a drug metabolism of the subject 504 affected by an interaction of the medication and a further medication used by the subject 504; determining the tolerance to the medication based on the drug-drug interaction information.
Clause 52. The method according to any one of the preceding clauses 45-47, comprising: receiving further medication information 106 representative of a further medication used by the subject 504, wherein the further medication does not influence the pathophysiological trait of the subject 504,wherein the further medication information 106 comprises information of a tolerance to the further medication; determining the tolerance to the medication based on the information of the tolerance to the further medication.
Clause 53. The method according to clause 52, wherein the further medication information 106 comprises information of the tolerance of the subject 504 to the further medication.
Clause 54. The method according to clause 52 or 53, wherein the further medication information 106 comprises information of the tolerance of a group of subjects to the further medication.
Clause 55. The method according to any one of clauses 52-54, wherein the medication and the further medication are in the same category.

### MEDICATION INTAKE

Clause 56. The method according to any one of the preceding clauses, comprising: wherein the medication information 106 comprises medication intake information representative of a medication intake of the subject 504, the method comprises: determining the setting 108 based on the medication intake information.
Clause 57. The method according to clause 56, comprising: generating a message signal adapted to display on a user interface a message relating to a request for the medication intake information, receiving a user interface input via the user interface in response to the message, determining the setting 108 based on the user interface input.
Clause 58. The method according to clause 57, comprising: receiving the medication intake information from a medication dispenser; detecting whether the medication intake information from the medication dispenser is inconsistent with the medication intake information from the user interface input; generating an alarm signal in case the medication intake information from the medication dispenser is inconsistent with the medication intake information from the user interface input.
Clause 59. The method according to clause 56 or 58, comprising: determining whether at least one prescribed medication intake did not occur based on the medication intake information; determining, in response to determining at least one prescribed medication intake did not occur, whether the influence of the medication on the pathophysiological trait is below a threshold level; determining a change in the setting 108 in response to the influence being below the threshold level, determining no change in the setting 108 in response to the influence not being below the threshold level.
Clause 60. The method according to any one of clauses 56- 59, wherein the medication intake information comprises information about a medication intake time, wherein the method comprises: determining the setting 108 of the therapy device based on the medication intake time.
Clause 61. The method according to any one of clauses 56-60, wherein the medication information 106 comprises a medication intake history representative of a history of an intake of the medication by the subject 504 during a period in the past, wherein the method comprises: estimating an effectiveness of the medication based on the medication intake history; determining the setting 108 of the therapy device based on the effectiveness of the medication.
Clause 62. The method according to any one of clauses 56-61, comprising: receiving the medication intake information from a medication dispenser.
Clause 63. The method according to any one of clauses 56-62, wherein the medication information 106 is indicative of a first medication and a second medication used by the subject 504, wherein the method comprises: determining a first setting of the therapy device based on the trait information representative of the influence of the first medication on the pathophysiological trait; determining a second setting of the therapy device based on the trait information representative of the influence of the second medication on the pathophysiological trait; determining the setting of the therapy device based on the first setting and the second setting.
Clause 64. The method according to clause 63, comprising: determining a desired effect profile for at least one of the first medication and the second medication based on the first setting 108 and the second setting 108, wherein the desired effect profile is representative of a desired effect during a sleep session of at least one of the first medication and the second medication; generating a profile output signal based on the desired effect profile.

### METHOD RELATING MAD

Clause 65. The method according to any one of the preceding clauses, wherein the therapy device comprises a mandibular advancement device (MAD 600), wherein the MAD 600 comprises a first member 601, a second member 602, and an adjustment device 604, wherein the first member 601 is adapted to engage a maxilla of the subject 504, wherein the second member 602 is adapted to engage a mandible of the subject 504, wherein the adjustment device 604 is adapted to adjust an offset 606 between the first member 601 and the second member 602 to advance the mandible relative to the maxilla, and wherein the method comprises: generating an instruction signal representative of an instruction for adjusting the offset 606 with the adjustment device 604, wherein the instruction signal is based on determining the setting 108 of the therapy device based on the trait information.
Clause 66. The method according to clause 65, wherein the adjustment device 604 comprises an actuator 608, wherein the method comprises: providing the instruction signal to the actuator 608, wherein the actuator 608 is adapted to adjust the offset 606 between the first member 601 and the second member 602 based on the instruction signal.
Clause 67. A computer program product, comprising instructions which, when executed by a processor, causes to carry out a computer-implemented method comprising: generating a message signal adapted to display on a user interface a message relating to a request for medication intake information, wherein the medication intake information is representative of a medication intake of the subject 504;
   receiving a user interface input via the user interface in response to the message; providing the user interface input as medication information 106 for use in the method according to any one of the preceding clauses.

### DEVICES

Clause 68. A computer program product, comprising instructions which, when executed by a processor, causes the method of any one of the clauses 1-66 to be carried out.
Clause 69. A computer-readable storage medium comprising instructions which, when executed by a processor, causes the method of any one of the clauses 1-66 to be carried out.
Clause 70. A respiratory support device 500 adapted to provide a pressurized airflow 502 to a subject 504, comprising: an air pressure source 506 adapted to generate the pressurized airflow 502; an outlet connectable to a patient interface to provide the pressurized airflow 502 to the subject 504; and a processor configured to execute a computer program product comprising instructions which, when executed by the processor, causes the method of clauses 1-66 to be carried out, wherein the respiratory support device 500 is the therapy device.
Clause 71. The respiratory support device 500 according to clause 70, wherein the setting 108 comprises a setting 108 of the air pressure source 506, wherein the processor is configured to control the air pressure source 506 to set the pressurized airflow 502 based on the setting 108.
Clause 72. A mandibular advancement device (MAD 600) comprising: a first member 601 adapted to engage a maxilla of the subject 504; a second member 602 adapted to engage a mandible of the subject 504; an adjustment device 604 adapted to adjust an offset 606 between the first member 601 and the second member 602 to advance the mandible relative to the maxilla, wherein the adjustment device 604 comprises an actuator 608, wherein the actuator 608 is adapted to receive the instruction signal generated according to the method of clause 65 or 66, wherein the MAD 600 is the therapy device, wherein the actuator 608 is adapted to adjust the offset 606 between the first member 601 and the second member 602 based on the instruction signal.
Clause 73. A positional therapy device 700, comprising: a body position sensor 800 adapted to generate a positional signal 806 representative of a body position of the subject 504; a stimulator adapted to provide a stimulus to the subject 504; a controller 804 configured to control the stimulator to provide the stimulus to the subject 504 in response to the positional signal 806 being representative of the body position being in a supine position; wherein the controller 804 comprises a processor configured to execute a computer program product comprising instructions which, when executed by the processor, causes the method of any one of clauses 1-66 to be carried out, wherein the positional therapy device 700 is the therapy device.
Clause 74. The positional therapy device 700 according to clause 73, wherein the setting 108 comprises a setting 108 of the stimulator, wherein the controller 804 is configured to set an intensity level of the stimulus provided by the stimulator based on the setting 108.

## Claims

1. A computer-implemented method for determining a setting (108) of a therapy device adapted to provide sleep disordered breathing (SDB) therapy to a subject (504), the method comprising:
receiving medication information (106) representative of medication used by the subject (504);
determining trait information based on the medication information (106),
wherein the trait information is representative of an influence of the medication on a pathophysiological trait (901-903) of the subject (504),
wherein the pathophysiological trait has an influence on the SDB of the subject (504); and
determining the setting (108) of the therapy device (500, 600, 700) based on the trait information.

2. The method according to claim 1, wherein the medication information (106) comprises information about a half-life of the medication, or a dosage of the medication, or a tolerance to the medication, or an effectiveness of the medication, or a duration of use of the medication, or a pharmacokinetic (PK) model, or a pharmacodynamic (PD) model, or a pharmacokinetic/ pharmacodynamic (PK/PD) model.

3. The method according to any one of the preceding claims, comprising
determining trait information during a first time period (210) and during a second time period (220),
wherein during the first time period the influence of the medication on the pathophysiological trait is larger than during the second time period;
determining the setting (108) of the therapy device to provide the SDB therapy having a therapy parameter, wherein the therapy parameter has a first value during the first time period and has a second value during the second time period, wherein the first value is different from the second value.

4. The method according to claim 3, wherein the therapy parameter relates to at least one of:
an intensity level of the SDB therapy;
a rate of change of the intensity level of the SDB therapy;
a range of the intensity level of the SDB therapy; or
a difference in intensity level of the SDB therapy between inhalation and exhalation of the subject (504).

5. The method according to claim 3 or 4, adjusting the therapy device according to the setting (108) to provide the SDB therapy having the first value during the first time period and having the second value during the second time period.

6. The method according to any one of claims 3-5,
wherein the first time period and the second time period are in a same sleep session of the subject (504), or
wherein the first time period and the second time period are in different sleep sessions of the subject (504).

7. The method according to any one of the preceding claims,
wherein the pathophysiological trait comprises a high pharyngeal collapsibility.

8. The method according to claim 7,
wherein the medication comprises a diuretic,
wherein the method comprises:
estimating a fluid shift in the subject (504) during a sleep session caused by the diuretic based on the medication information (106)
determining the trait information based on the fluid shift.

9. The method according to claim 8, comprising:
estimating a urinary time period in the sleep session based on the medication information (106),
wherein during the urinary time period the subject (504) has likely a need to urinate;
determining the setting (108) of the therapy device to provide the SDB therapy at a lower intensity level during the urinary time period than before the urinary time period.

10. The method according to any one of claims 8-9, comprising
receiving blood pressure information (1906) representative of a blood pressure of the subject (504);
determining the trait information based on the blood pressure information (1906).

11. The method according to any one of claims 7-10, wherein the medication comprises a weight loss medication,
wherein the method comprises providing a weight loss estimation based on the medication information (106); and
determining the trait information based on the weight loss estimation.

12. The method according to any one of claims 7-11, comprising:
receiving neck circumference information or body weight information representative of a body weight of the subject (504);
determining the trait information based on the neck circumference information or the body weight information.

13. A computer program product, comprising instructions which, when executed by a processor, causes the method of any one of the claims 1-12 to be carried out.

14. A computer-readable storage medium comprising instructions which, when executed by a processor, causes the method of any one of the claims 1-12 to be carried out.

15. A respiratory support device (500) adapted to provide a pressurized airflow (502) to a subject (504), comprising:
an air pressure source (506) adapted to generate the pressurized airflow (502);
an outlet connectable to a patient interface to provide the pressurized airflow (502) to the subject (504); and
a processor configured to execute a computer program product comprising instructions which, when executed by the processor, causes the method of claims 1-12 to be carried out,
wherein the respiratory support device (500) is the therapy device.
